# EUROPEAN PATENT APPLICATION

(11) **EP 4 644 418 A1**
(43) Date of publication of application: **05.11.2025**
(21) Application number: 23908853.7
(22) Date of filing: 21.03.2023
(51) Int. Cl.: C07K 16/28, C07K 19/00, C12N 15/13, C12N 15/62, C12N 15/867, C12N 5/10, A61P 35/00, A61P 35/02, A61K 47/68, A61K 39/00, G01N 33/68, G01N 33/574

(54) **NANOBODY TARGETING BCMA, CHIMERIC ANTIGEN RECEPTOR AND USE THEREOF**

(30) Priority: 26.12.2022 CN 202211679187
(71) Applicant: Hebei Senlang Biotechnology Co. Ltd., Shijiazhuang, Hebei 050000 (CN)
(72) Inventor: LI, Jianqiang, Shijiazhuang, Hebei 050000 (CN); WANG, Lin, Shijiazhuang, Hebei 050000 (CN); ZHAO, Na, Shijiazhuang, Hebei 050000 (CN)
(74) Representative: IPTector Consulting ApS
(86) International application number: PCT/CN2023/082897
(87) International publication number: WO 2024/138897

(57) **Abstract**

Provided are a nanobody targeting BCMA and a chimeric antigen receptor thereof. The nanobody comprises VHH01 and VHH02, and has strong specificity and high affinity for BCMA. A constructed CAR-T cell has high specific killing efficiency on a BCMA positive cell line, and can be used for developing an immunotherapeutic drug targeting BCMA.

## Description

### CROSS-REFERENCE TO RELATED APPLICATIONS

The present application claims priority to Chinese Patent Application No. 2022116791873 entitled "NANOBODY TARGETING BCMA, CHIMERIC ANTIGEN RECEPTOR AND USE THEREOF" and filed December 26, 2022, the disclosure of which is incorporated herein by reference.

### FIELD OF THE INVENTION

This application relates to the technical field of biological medicine, specifically to a nanobody targeting BCMA, a chimeric antigen receptor and a use thereof, and more specifically to nanobodies VHH01 and VHH02 targeting BCMA, a chimeric antigen receptor and a use thereof.

### BACKGROUND

B cell maturation antigen (BCMA) is an antigen expressed in plasma cells, plasmablasts, and bone marrow plasma cells, and is not expressed on B cells or hematopoietic stem cells. BCMA, also known as CD269 and TNFRSF17, belongs to the TNF receptor superfamily, is a type III transmembrane protein composed of 185 amino acid residues, and can bind B lymphocyte stimulating factor (BAFF) and proliferation-inducing ligand (APRIL). The expression of BCMA is associated with many cancers, autoimmune diseases, and infectious diseases. Relevant cancers with high expression of BCMA include some hematological cancers, such as multiple myeloma, Hodgkin's lymphoma, non-Hodgkin's lymphoma, various leukemias, glioblastoma, etc. In addition, BCMA plays a key role in cell survival, proliferation, metastasis, and drug resistance by mediating downstream signaling pathways. These properties make BCMA an effective target for the treatment of hematological tumors, especially for the treatment of multiple myeloma. At present, new tumor immunotherapy methods for BCMA mainly include Chimeric Antigen Receptor T-Cell Immunotherapy (CAR-T), Bispecific antibody (BsAb) drugs, and antibody-drug conjugates (ADC), recent studies have also shown that CAR-T cells targeting BCMA can effectively eliminate myeloma cells in vivo.

CAR-T is a new type of precision targeted therapy for the treatment of tumors. Its principle is that T cells modified by chimeric antigen receptor (CAR) can specifically recognize tumor-associated antigens on the surface of tumor cells, making the targeting, killing activity, and persistence of effector T cells higher than those of conventionally used immune cells, and can overcome the local immunosuppressive microenvironment of tumors and break the immune tolerance state of the host. CAR is the core structure of CAR-T, which endows cells with the ability to specifically recognize tumor-associated antigens on the surface of tumor cells. CAR is mainly composed of three functional domains: an extracellular domain, a transmembrane domain, and an intracellular domain. The extracellular domain is composed of an antigen-binding region and a hinge region that provides a connection effect. The intracellular domain is composed of a costimulatory domain and a signaling domain. After the extracellular antigen binding region binds to the target protein expressed on the surface of the target cell, it will activate the costimulatory domain and signal transduction domain of the CAR structure, so that CAR-T has both activation signal and co-activation signal, and can achieve efficient expansion while killing tumor cells. The effectiveness of CAR-T therapy depends on properties such as the specificity of the antibody that recognizes the antigen and the high or low affinity of the antibody to bind to the antigen.

At present, CAR T therapy generally uses scFv segments derived from monoclonal antibodies as antigen binding regions, but scFv has a large molecular weight and is likely to form multimers, affecting the function of CAR and the therapeutic effect of CAR T cells. Nanobodies are currently the smallest antibody molecules. Since their discovery, nanobodies have gradually attracted people's attention. Nanobodies have been used in autoimmune diseases, blood diseases, virus infection-related diseases, and orthopedic diseases, and have also shown great advantages in anti-infection and anti-inflammatory diseases and anti-neurodegenerative diseases. Main advantages of nanobodies are as follows. 1. Small molecular weight and strong tissue penetration ability. The size of nanobodies is only 1/10 of that of ordinary antibodies, so nanobodies have strong tissue penetration ability and can even directly penetrate the blood-brain barrier, thereby improving the therapeutic effect on solid tumors. 2. Low toxicity and immunogenicity. Due to their small molecular weight and small size, nanobodies have low immunogenicity and toxicity to the host. 3. High affinity and strong stability. Nanobodies have high affinity with antigens, are stable, soluble in water, and also have strong acid and alkali resistance. 4. Diverse combination forms. Nanobodies can be made into single, double, and multifunctional antibodies to achieve simultaneous attack on multiple targets. 5. Short production cycle and low production cost. Nanobodies are easy to prepare, and can be made into drugs in one to two years after the technology is stable. 6. Wide range of applications. Nanobodies can be widely used in disease treatment, medical diagnosis, immunoaffinity chromatography, and other fields. To overcome the above technical problems in conventional CAR-T therapy and make full use of the above advantages of nanobodies, the present invention provides novel BCMA-targeting nanobodies VHH01 and VHH02, and CAR-T cells comprising the nanobodies.

### SUMMARY

An objective of the present application is to overcome the shortcomings in the prior art and provide a nanobody targeting BCMA, a chimeric antigen receptor, and a use thereof. The nanobody comprises VHH01 or VHH02. Both VHH01 and VHH02 have strong specificity and high affinity for BCMA. CAR-T cells prepared based on the nanobody have high specific killing efficiency on a BCMA positive cell line, have no adverse effects on cell lines that do not express BCMA, and have broad application prospects. On this basis, the following invention was completed.

### Nanobody

According to one aspect, the present application provides a nanobody targeting BCMA.

Further, the nanobody comprises VHH01 or VHH02, wherein
amino acid sequences of CDR1, CDR2, and CDR3 of the VHH01 are as shown in SEQ ID NO: 3, SEQ ID NO: 5, and SEQ ID NO: 7, respectively; amino acid sequences of CDR1, CDR2, and CDR3 of the VHH02 are as shown in SEQ ID NO: 11, SEQ ID NO: 13, and SEQ ID NO: 15, respectively.

Further, nucleotide sequences of the CDR1, the CDR2, and the CDR3 of the VHH01 are as shown in SEQ ID NO: 4, SEQ ID NO: 6, and SEQ ID NO: 8, respectively. Further, an amino acid sequence of the VHH01 is as shown in SEQ ID NO: 1. Further, a nucleotide sequence of the VHH01 is as shown in SEQ ID NO: 2.

Further, nucleotide sequences of the CDR1, CDR2, and CDR3 of the VHH02 are as shown in SEQ ID NO: 12, SEQ ID NO: 14, and SEQ ID NO: 16, respectively. Further, the amino acid sequence of the VHH02 is as shown in SEQ ID NO: 9. Further, a nucleotide sequence of the VHH02 is as shown in SEQ ID NO: 10.

Any nanobody corresponding to the amino acid sequence or nucleotide sequence that has at least 75% identity with the above amino acid sequences or nucleotide sequences of the present application, *i.e.,* an amino acid sequence or nucleotide sequence obtained by replacing amino acids or nucleotides at any one or more positions with any other amino acids or nucleotides on the basis of the corresponding amino acid sequences or nucleotide sequences, falls within the scope of protection of the present application.

In some embodiments, the nanobody further comprises a functional variant of nanobody VHH01 or a functional variant of nanobody VHH02. Specifically, the functional variant comprises, but is not limited to, derivatives that are substantially similar in primary structural sequence, but comprise chemical and/or biochemical modifications not present in the parent nanobody VHH01 or VHH02 of the present application, *e.g*. in vitro or in vivo. These modifications comprise, for example, acetylation, acylation, covalent attachment of nucleotides or nucleotide derivatives, covalent attachment of lipids or lipid derivatives, crosslinking, formation of disulfide bonds, glycosylation, hydroxylation, methylation, oxidation, pegylation, proteolytic treatment, or phosphorylation, and such functional variants also fall within the scope of protection of the present application.

Optionally, the functional variant may be the following nanobody: comprising an amino acid sequence containing substitution, insertion, deletion, or a combination thereof of one or more amino acids as compared to the amino acid sequence of the parent nanobody VHH01 or VHH02. Further, the functional variant may comprise a truncation of the amino acid sequence at one or both of the amino-terminus or carboxy-terminus. The functional variant of the present application may have the same or different, higher or lower binding affinity as compared to the parent nanobody VHH01 or VHH02, but still be able to specifically bind to BCMA. For example, the functional variant of the present application may have an increased or decreased binding affinity for BCMA as compared to the parent nanobody VHH01 or VHH02.

In the present application, the term "identity" indicates that at any particular position of the aligned sequences, the amino acid residues between the sequences are the same. As used herein, "similarity" indicates that at any particular position of the aligned sequences, the amino acid residues between the sequences are of similar type. For example, leucine can be replaced with isoleucine or valine. Other amino acids that are commonly substituted for each other comprise, but are not limited to: phenylalanine, tyrosine, and tryptophan (amino acids with aromatic side chains); lysine, arginine, and histidine (amino acids with basic side chains); aspartic acid and glutamic acid (amino acids with acidic side chains); asparagine and glutamine (amino acids with amide side chains); and cysteine and methionine (amino acids with sulfur-containing side chains). Generally, modification of one or more amino acids in a protein does not affect the function of the protein. Those skilled in the art will recognize that changing a single amino acid or a small percentage of amino acids or individual additions, deletions, insertions, or substitutions to an amino acid sequence are conservative modifications, where changes in proteins produce proteins with similar functions, and conservative substitution tables providing amino acids with similar functions is well known in the art.

In the present application, the term "conservative modification" refers to an amino acid modification that does not significantly affect or change the binding characteristics of the nanobody containing the amino acid sequence. Such conservative modifications comprise amino acid substitutions, additions, and deletions. Modifications can be introduced into the nanobody of the present application by standard techniques known in the art (such as site-directed mutagenesis and PCR-mediated mutagenesis). Conservative substitutions are substitutions in which an amino acid residue is replaced by an amino acid residue with a similar side chain. Families of amino acid residues with similar side chains have been defined in the art. These families comprise amino acids with basic side chains (*e.g.,* lysine, arginine, or histidine), acidic side chains (*e.g.,* aspartic acid or glutamic acid), uncharged polar side chains (*e.g.,* glycine, asparagine, glutamine, serine, threonine, tyrosine, cysteine, or tryptophan), nonpolar side chains (*e.g.,* alanine, valine, leucine, isoleucine, proline, phenylalanine, or methionine), β-branched side chains (*e.g.,* threonine, valine, and isoleucine), and aromatic side chains (*e.g.,* tyrosine, phenylalanine, tryptophan, or histidine).

In the present application, the term "BCMA" is used interchangeably with "CD269" and "TNFRSF17", and generally refers to B cell maturation antigen. For example, human BCMA is generally a 184 amino acid long protein encoded by a 994 nucleotide long primary mRNA transcript (NM_001192.2). The amino acid sequence of human BCMA is represented by UniProtKB accession number Q02223. In the present application, the term "BCMA" may comprise proteins comprising mutations, for example, proteins comprising point mutations, fragments, insertions, deletions, and splice variants of full-length wild-type BCMA. In the present application, the term "BCMA" may also comprise a portion of the complete BCMA protein as long as the relevant biological activity is retained.

### Single-nanobody-based chimeric antigen receptor

According to another aspect, the present application provides a single-nanobody-based chimeric antigen receptor targeting BCMA.

Further, the chimeric antigen receptor comprises the nanobody according to any one of the embodiments of the first aspect of the present application. Further, the chimeric antigen receptor further comprises a transmembrane domain. Further, the chimeric antigen receptor further comprises an intracellular signaling domain. Further, the chimeric antigen receptor further comprises a hinge region. Further, the chimeric antigen receptor further comprises a signal peptide. Further, the chimeric antigen receptor further comprises a costimulatory domain. Further, the chimeric antigen receptor further comprises a promoter. Further, the chimeric antigen receptor further comprises a self-cleaving peptide. Further, the chimeric antigen receptor further comprises a detection tag/auxiliary function element. Further, the chimeric antigen receptor further comprises a tEGFR signal peptide.

Further, the transmembrane domain comprises a transmembrane domain of the following molecules: CD8α, CD28, 4-1BB, CD34, CD3ε, PD-1, IgG1, IgG4, OX40, IL-2 receptor, IL-7 receptor, or IL-11 receptor. Further, the intracellular signaling domain comprises an intracellular signaling domain of the following molecules: CD3ζ, CD3γ, CD3δ, CD3ε, FcRγ, FcRβ, TCRζ, CD4, CD5, CD8, CD21, CD22, CD79a, CD79b, CD278, FcεRI, DAP10, DAP12, or CD66d. Further, the hinge region comprises a hinge region of the following molecules: CD8α, CD28, CD34, 4-1BB, OX40, CD3ε, IgG1, IgG4, PD-1, IL-2 receptor, IL-7 receptor, or IL-11 receptor. Further, the signal peptide comprises a signal peptide of the following molecules: T cell receptor α- and β- chains, CD3ζ, CD3ε, CD16, CD22, CD33, CD4, CD5, CD8, CD9, CD28, CD37, CD45, CD64, CD80, CD86, CD134, CD137, CD154, GITR, or GM-CSF. Further, the costimulatory domain comprises a costimulatory domain of the following molecules: 4-1BB(CD137), CD19, CD4, CD27, CD28, ICOS(CD278), CD8α, CD8β, BAFFR, HVEM, LIGHT, KIRDS2, SLAMF7, NKp30, NKp46, CD40, CDS, ICAM-1, B7-H3, OX40, DR3, GITR, CD30, TIM1, CD2, CD7, or CD226. Further, the promoter comprises: EF1α promoter, CMV promoter, EFS promoter, CAG promoter, CBh promoter, SFFV promoter, MSCV promoter, SV40 promoter, mPGK promoter, hPGK promoter, or UBC promoter. Further, the self-cleaving peptide comprises: T2A, P2A, E2A, or F2A. Further, the detection tag/auxiliary function element comprises: tEGFR, tCD34, tCD19, tCD20, tCD22, immune checkpoint inhibitor (CTLA-4, PD-1/PD-L1, LAG-3, TIM-3, TIGIT, CD226, CD155, CD47, B7-H3, or B7-H4) nanobodies, cytokines or receptors thereof (IL2, IL2 receptor, IL7, IL7 receptor, IL15, or IL15 receptor).

Further, the transmembrane domain is a CD8a transmembrane domain. Further, the intracellular signaling domain is a CD3ζ intracellular signaling domain. Further, the hinge region is a CD8α hinge region. Further, the costimulatory domain is a 4-1BB costimulatory domain. Further, the promoter is EF1α. Further, the self-cleaving peptide is T2A. Further, the detection tag/auxiliary function element is tEGFR.

Further, the chimeric antigen receptor is obtained by connecting EF1α, a signal peptide, the nanobody according to any one of the embodiments of the first aspect of the present application, a CD8a hinge region, a CD8a transmembrane domain, a 4-1BB costimulatory domain, a CD3ζ intracellular signaling domain, T2A, a tEGFR signal peptide, and tEGFR sequentially.

Further, the chimeric antigen receptor is obtained by connecting EF1α, a signal peptide, VHH01, a CD8α hinge region, a CD8a transmembrane domain, a 4-1BB costimulatory domain, a CD3ζ intracellular signaling domain, T2A, a tEGFR signal peptide, and tEGFR sequentially.

Further, the chimeric antigen receptor is obtained by connecting EF1α, a signal peptide, VHH02, a CD8a hinge region, a CD8a transmembrane domain, a 4-1BB costimulatory domain, a CD3ζ intracellular signaling domain, T2A, a tEGFR signal peptide, and tEGFR sequentially.

In some embodiments, a nucleotide sequence of the EF1α in the present application is as shown in SEQ ID NO: 17, amino acid sequences of the signal peptide, the CD8α hinge region, the CD8α transmembrane domain, the 4-1BB costimulatory domain, the CD3ζ intracellular signaling domain, the T2A, the tEGFR signal peptide, and the tEGFR are respectively as shown in SEQ ID NO: 18 to SEQ ID NO: 25, and nucleotide sequences of the signal peptide, the CD8a hinge region, the CD8a transmembrane domain, the 4-1BB costimulatory domain, the CD3ζ intracellular signaling domain, the T2A, the tEGFR signal peptide, and the tEGFR are respectively as shown in SEQ ID NO: 26 to SEQ ID NO: 33.

In some embodiments, the EF1α, the signal peptide, the CD8a hinge region, the CD8a transmembrane domain, the 4-1BB costimulatory domain, the CD3ζ intracellular signaling domain, the T2A, the tEGFR signal peptide, and the tEGFR in the present application are not limited to the above sequences, and any amino acid sequence or nucleotide sequence having at least 75% identity with the above amino acid sequences or nucleotide sequences shall fall within the scope of protection of the present application.

In the present application, the term "chimeric antigen receptor (CAR)" generally refers to a fusion protein comprising an extracellular domain capable of binding to an antigen and at least one intracellular domain. CAR is a core component of a chimeric antigen receptor T cell (CAR-T), which may comprise an antigen (*e.g.,* tumor-specific antigen and/or tumor-associated antigen) binding domain, a transmembrane domain, a costimulatory domain, and an intracellular signaling domain. CAR is an engineered receptor that can implant any specific receptor onto immune effector cells, especially T cells. In CAR, a VHH fragment that specifically recognizes a tumor antigen or a scFv fragment of a monoclonal antibody can be implanted onto a T cell or NK cell. A nucleic acid encoding CAR can be introduced into a T cell, NK cell, or NK T cell using, for example, a retroviral vector. In this way, a large number of cancer-specific T cells, NK cells, or NK T cells can be generated for adoptive cell transfer. In the present application, the CAR can be combined with a T cell receptor activation intracellular domain based on the antigen (*e.g.,* BCMA) specificity of the antibody. T cells genetically modified to express CAR can specifically recognize and eliminate malignant cells expressing the target antigen.

### Dual-nanobody-based chimeric antigen receptor

According to another aspect, the present application provides a dual-nanobody-based chimeric antigen receptor targeting BCMA. The chimeric antigen receptor comprises the nanobody according to any one of the embodiments of the first aspect of the present application and any another nanobody targeting BCMA.

Further, the any another nanobody targeting BCMA is nanobody VHH02. Further, amino acid sequences of CDR1, CDR2, and CDR3 of the VHH02 are as shown in SEQ ID NO: 11, SEQ ID NO: 13, and SEQ ID NO: 15, respectively. Further, nucleotide sequences of the CDR1, the CDR2, and the CDR3 of the VHH02 are as shown in SEQ ID NO: 12, SEQ ID NO: 14, and SEQ ID NO: 16, respectively. Further, an amino acid sequence of the VHH02 is as shown in SEQ ID NO: 9. Further, a nucleotide sequence of the VHH02 is as shown in SEQ ID NO: 10.

In some embodiments, the nanobody VHH02 of the present application further comprises a nanobody corresponding to an amino acid sequence or nucleotide sequence that has at least 75% identity with the above amino acid sequences or nucleotide sequences of the present application, *i.e.,* any amino acid sequence or nucleotide sequence obtained by replacing amino acids or nucleotides at any one or more positions with any other amino acids or nucleotides on the basis of the corresponding amino acid sequences or nucleotide sequences falls within the scope of protection of the present application as long as the amino acid sequence or nucleotide sequence has at least 75% identity with the above amino acid sequences or nucleotide sequences of the present application.

Further, the chimeric antigen receptor further comprises a transmembrane domain. Further, the chimeric antigen receptor further comprises an intracellular signaling domain. Further, the chimeric antigen receptor further comprises a hinge region. Further, the chimeric antigen receptor further comprises a signal peptide. Further, the chimeric antigen receptor further comprises a costimulatory domain. Further, the chimeric antigen receptor further comprises a promoter. Further, the chimeric antigen receptor further comprises a self-cleaving peptide. Further, the chimeric antigen receptor further comprises a detection tag/auxiliary function element. Further, the chimeric antigen receptor further comprises a tEGFR signal peptide.

Further, the transmembrane domain comprises a transmembrane domain of the following molecules: CD8α, CD28, 4-1BB, CD34, CD3ε, PD-1, IgG1, IgG4, OX40, IL-2 receptor, IL-7 receptor, or IL-11 receptor. Further, the intracellular signaling domain comprises an intracellular signaling domain of the following molecules: CD3ζ, CD3γ, CD3δ, CD3ε, FcRγ, FcRβ, TCRζ, CD4, CD5, CD8, CD21, CD22, CD79a, CD79b, CD278, FcεRI, DAP10, DAP12, or CD66d. Further, the hinge region comprises a hinge region of the following molecules: CD8α, CD28, CD34, 4-1BB, OX40, CD3ε, IgG1, IgG4, PD-1, IL-2 receptor, IL-7 receptor, or IL-11 receptor. Further, the signal peptide comprises a signal peptide of the following molecules: T cell receptor α- and β- chains, CD3ζ, CD3ε, CD16, CD22, CD33, CD4, CD5, CD8, CD9, CD28, CD37, CD45, CD64, CD80, CD86, CD134, CD137, CD154, GITR, or GM-CSF. Further, the costimulatory domain comprises a costimulatory domain of the following molecules: 4-1BB(CD137), CD19, CD4, CD27, CD28, ICOS(CD278), CD8α, CD8β, BAFFR, HVEM, LIGHT, KIRDS2, SLAMF7, NKp30, NKp46, CD40, CDS, ICAM-1, B7-H3, OX40, DR3, GITR, CD30, TIM1, CD2, CD7, or CD226. Further, the promoter comprises: EF1α promoter, CMV promoter, EFS promoter, CAG promoter, CBh promoter, SFFV promoter, MSCV promoter, SV40 promoter, mPGK promoter, hPGK promoter, or UBC promoter. Further, the self-cleaving peptide comprises: T2A, P2A, E2A, or F2A. Further, the detection tag/auxiliary function element comprises: tEGFR, tCD34, tCD19, tCD20, tCD22, immune checkpoint inhibitor (CTLA-4, PD-1/PD-L1, LAG-3, TIM-3, TIGIT, CD226, CD155, CD47, B7-H3, or B7-H4) nanobodies, cytokines or receptors thereof (IL2, IL2 receptor, IL7, IL7 receptor, IL15, or IL15 receptor).

Further, the transmembrane domain is a CD8a transmembrane domain. Further, the intracellular signaling domain is a CD3ζ intracellular signaling domain. Further, the hinge region is a CD8a hinge region. Further, the costimulatory domain is a 4-1BB costimulatory domain. Further, the promoter is EF1α. Further, the self-cleaving peptide is T2A. Further, the detection tag/auxiliary function element is tEGFR.

Further, the chimeric antigen receptor is obtained by connecting EF1α, a signal peptide, the nanobody according to any one of the embodiments of the first aspect of the present application, a linker, any another nanobody targeting BCMA, a CD8a hinge region, a CD8a transmembrane domain, a 4-1BB costimulatory domain, a CD3ζ intracellular signaling domain, T2A, a tEGFR signal peptide, and tEGFR sequentially.

Further, the chimeric antigen receptor is obtained by connecting EF1α, a signal peptide, any another nanobody targeting BCMA, a linker, the nanobody according to any one of the embodiments of the first aspect of the present application, a CD8a hinge region, a CD8a transmembrane domain, a 4-1BB costimulatory domain, a CD3ζ intracellular signaling domain, T2A, a tEGFR signal peptide, and tEGFR sequentially.

Further, the chimeric antigen receptor is obtained by connecting EF1α, a signal peptide, VHH01, a linker, VHH02, a CD8a hinge region, a CD8a transmembrane domain, a 4-1BB costimulatory domain, a CD3ζ intracellular signaling domain, T2A, a tEGFR signal peptide, and tEGFR sequentially.

Further, the chimeric antigen receptor is obtained by connecting EF1α, a signal peptide, VHH02, a linker, VHH02, a CD8a hinge region, a CD8a transmembrane domain, a 4-1BB costimulatory domain, a CD3ζ intracellular signaling domain, T2A, a tEGFR signal peptide, and tEGFR sequentially.

Further, the linker is (G4S)₅.

In some embodiments, the linker is a linker conventionally used in the art, and the linker is not limited to (G4S)₅, and may be any one of (GGGGS) n, (GGGS) n, (SSSSG)n, (GSGSA)n, (GGSGG)n, or other linkers, where n may be any integer from 1 to 10.

In some embodiments, a nucleotide sequence of the EF1α in the present application is as shown in SEQ ID NO: 17, amino acid sequences of the signal peptide, the CD8a hinge region, the CD8a transmembrane domain, the 4-1BB costimulatory domain, the CD3ζ intracellular signaling domain, the T2A, the tEGFR signal peptide, and the tEGFR are respectively as shown in SEQ ID NO: 18 to SEQ ID NO: 25, and nucleotide sequences of the signal peptide, the CD8α hinge region, the CD8a transmembrane domain, the 4-1BB costimulatory domain, the CD3ζ intracellular signaling domain, the T2A, the tEGFR signal peptide, and the tEGFR are respectively as shown in SEQ ID NO: 26 to SEQ ID NO: 33.

In some embodiments, the EF1α, the signal peptide, the CD8a hinge region, the CD8a transmembrane domain, the 4-1BB costimulatory domain, the CD3ζ intracellular signaling domain, the T2A, the tEGFR signal peptide, and the tEGFR in the present application are not limited to the above sequences, and any amino acid sequence or nucleotide sequence having at least 75% identity with the above amino acid sequences or nucleotide sequences shall fall within the scope of protection of the present application.

In some embodiments, the chimeric antigen receptor in the present application may further comprise an alternative marker, such as a cell surface marker (*e.g.,* a truncated cell surface marker), which can be used to prove that the cell is transduced or engineered to express the receptor. In some aspects, the alternative marker comprises an entirety or part (*e.g.,* a truncated form) of CD34, NGFR, CD19, truncated CD19, or epidermal growth factor receptor (*e.g.,* tEGFR). In some other aspects, the cell surface marker comprises a fluorescent protein, *e.g.,* green fluorescent protein (GFP), enhanced green fluorescent protein (EGFP) such as superfolded GFP (sfGFP), red fluorescent protein (RFP) such as tdTomato, mCherry, mStrawberry, AsRed2, DsRed, or DsRed2, cyan fluorescent protein (CFP), blue fluorescent protein (BFP), enhanced blue fluorescent protein (EBFP),, yellow fluorescent protein (YFP), and variants thereof; and also comprises species variants, monomeric variants, and codon-optimized and/or enhanced variants of fluorescent proteins. In some other aspects, the alternative marker comprises an enzyme such as luciferase, lacZ gene from *Escherichia Coli* (*E. coli*)*,* alkaline phosphatase, secreted embryonic alkaline phosphatase (SEAP), or chloramphenicol acetyltransferase (CAT), and also comprises β-galactosidase, chloramphenicol acetyltransferase (CAT), β-glucuronidase (GUS), or variants thereof.

### Nucleic acid molecule

According to another aspect, the present application provides an isolated nucleic acid molecule.

Further, the nucleic acid molecule comprises a nucleotide sequence encoding the nanobody according to the first aspect of the present application, the chimeric antigen receptor according to the second aspect of the present application, or the chimeric antigen receptor according to the third aspect of the present application.

Further, a nucleotide sequence of the VHH01 in the nanobody according to the first aspect of the present application is as shown in SEQ ID NO: 2. Further, a nucleotide sequence of the VHH02 in the nanobody according to the first aspect of the present application is as shown in SEQ ID NO: 10. Further, a nucleotide sequence of EF1α in the chimeric antigen receptor according to the second aspect of the present application or the chimeric antigen receptor according to the third aspect of the present application is as shown in SEQ ID NO: 17. Further, a nucleotide sequence of the signal peptide in the chimeric antigen receptor according to the second aspect of the present application or the chimeric antigen receptor according to the third aspect of the present application is as shown in SEQ ID NO: 26. Further, a nucleotide sequence of the CD8α hinge region in the chimeric antigen receptor according to the second aspect of the present application or the chimeric antigen receptor according to the third aspect of the present application is as shown in SEQ ID NO: 27. Further, a nucleotide sequence of the CD8a transmembrane domain in the chimeric antigen receptor according to the second aspect of the present application or the chimeric antigen receptor according to the third aspect of the present application is as shown in SEQ ID NO: 28. Further, a nucleotide sequence of the 4-1BB costimulatory domain in the chimeric antigen receptor according to the second aspect of the present application or the chimeric antigen receptor according to the third aspect of the present application is as shown in SEQ ID NO: 29. Further, a nucleotide sequence of the CD3ζ intracellular signaling domain in the chimeric antigen receptor according to the second aspect of the present application or the chimeric antigen receptor according to the third aspect of the present application is as shown in SEQ ID NO: 30. Further, a nucleotide sequence of T2A in the chimeric antigen receptor according to the second aspect of the present application or the chimeric antigen receptor according to the third aspect of the present application is as shown in SEQ ID NO: 31. Further, a nucleotide sequence of the tEGFR signal peptide in the chimeric antigen receptor according to the second aspect of the present application or the chimeric antigen receptor according to the third aspect of the present application is as shown in SEQ ID NO: 32. Further, a nucleotide sequence of tEGFR in the chimeric antigen receptor according to the second aspect of the present application or the chimeric antigen receptor according to the third aspect of the present application is as shown in SEQ ID NO: 33.

In the present application, the term "nucleic acid molecule" refers to DNA molecules and RNA molecules. Nucleic acid molecules can be single-stranded or double-stranded, but are preferably double-stranded DNA. A nucleic acid is "operably linked" when it is placed in a functional relationship with another nucleic acid sequence. For example, if a promoter or enhancer affects the transcription of a coding sequence, the promoter or enhancer is operably linked to the coding sequence.

### Expression vector

According to another aspect, the present application provides an expression vector comprising the nucleic acid molecule according to the fourth aspect of the present application.

Further, the vector comprises a DNA vector, an RNA vector, a plasmid, and a vector derived from virus. Further, the vector derived from virus comprises a lentiviral vector, a retroviral vector, an adenoviral vector, an adeno-associated virus vector, a poxvirus vector, and a herpes virus vector.

In some embodiments, the vector is not particularly limited in the present application, and may be selected depending on a desired function. Non-limiting examples of the vector comprise a plasmid vector, a vector derived from virus, a bacteriophage vector, and other vectors conventionally used, for example, in genetic engineering. Various plasmids and vectors can be constructed based on methods well known to those skilled in the art.

In some embodiments, the expression vector according to the present application is capable of directing the replication and expression of the nucleic acid molecule of the present application in the host, and thus ensuring the expression of the nanobody targeting BCMA or the chimeric antigen receptor of the present application encoded thereby in the selected host. The expression vector may be, for example, a cloning vector, a binary vector, or an integrative vector. Expression comprises transcription of the nucleic acid molecule, for example, transcription into translatable mRNA.

Non-limiting examples of the vector comprise pQE-12, pUC-series, pBluescript (Stratagene), pET-series expression vectors (Novagen) or pCRTOPO (Invitrogen), λgt11, pJOE, pBBR1-MCS series, pJB861, pBSMuL, pBC2, pUCPKS, pTACT1, pTRE, pCAL-n-EK, pESP-1, pOP13CAT, E-027 pCAGKosak-Cherry (L45a) vector system, pREP (Invitrogen), pCEP4 (Invitrogen), pMC1neo(Stratagene), pXT1 (Stratagene), pSG5 (Stratagene), EBO-pSV2neo, pBPV-1, pdBPVMMTneo, pRSVgpt, pRSVneo, pSV2-dhfr, pIZD35, Okayama-Berg cDNA expression vector pcDV1 (Pharmacia), pRc/CMV, pcDNA1, pcDNA3 (Invitrogen), pcDNA3.1, pSPORT1 (GIBCO BRL), pGEMHE (Promega), pLXIN, pSIR (Clontech), pIRES-EGFP (Clontech), pEAK-10 (EdgeBiosystems) pTriEx-Hygro (Novagen), and pCINeo (Promega). Non-limiting examples of plasmid vectors suitable for *Pichia pastoris* comprise, for example, plasmids pAO815, pPIC9K, and pPIC3.5K (all from Invitrogen). Another vector suitable for expressing proteins in Xenopus embryos, zebrafish embryos, and a wide variety of mammalian and avian cells is the multipurpose expression vector pCS2+.

Generally, a vector may contain one or more origins of replication (ori) and genetic systems for cloning or expression, one or more markers for selection in a host (*e.g.,* antibiotic resistance), and one or more expression cassettes. In addition, the coding sequence contained in the vector may be linked to transcriptional regulatory elements and/or to other amino acid coding sequences using established methods. Such regulatory sequences are well known to those skilled in the art and comprise, but are not limited to, regulatory sequences ensuring transcription initiation, internal ribosome entry sites (IRES), and optional regulatory elements ensuring transcription termination and transcript stability. Non-limiting examples of such regulatory elements ensuring initiation of transcription comprise promoters, translation start codons, enhancers, insulators, and/or regulatory elements ensuring termination of transcription, which are comprised downstream of the nucleic acid molecule of the present application. Further examples comprise Kozak sequences and intervening sequences flanked by donor and acceptor sites for RNA splicing, nucleotide sequences encoding secretion signals, or signal sequences depending on the expression system used, which are capable of directing the expressed protein to a cell compartment or culture medium. The vector may also contain additional expressible polynucleotides encoding one or more protein chaperones to promote correct protein folding.

In the present application, the term "expression vector" refers to a vector comprising a recombinant polynucleotide, which comprises an expression control sequence operably linked to a nucleotide sequence to be expressed. The expression vector comprises sufficient cis-acting elements for expression. Other elements for expression may be provided by the host cell or in an in-vitro expression system. Expression vectors comprise all those known in the art, comprising cosmids, plasmids (*e.g.,* naked or contained in liposomes) and viruses (*e.g.,* lentiviruses, retroviruses, adenoviruses, or adeno-associated viruses) into which the recombinant polynucleotide is incorporated.

### Engineered host cell

According to another aspect, the present application provides an engineered host cell.

Further, the engineered host cell comprises the expression vector according to the fifth aspect of the present application. Further, the host cell is a eukaryotic cell or a prokaryotic cell. Further, the host cell is an immune cell. Further, the immune cell comprises T cells, B cells, NK cells, iNKT cells, CTL cells, dendritic cells, myeloid cells, monocytes, macrophages, gdT cells, any immune cells derived from iPS, or any combination thereof. Further, the immune cell is a T cell.

In the present application, the term "host cell" refers to a cell that can be used to introduce a vector, comprising but not limited to: prokaryotic cells such as *Escherichia coli* or *Bacillus subtilis,* fungal cells such as yeast cells or *Aspergillus,* insect cells such as *Drosophila* S2 cells or Sf9, or animal cells such as fibroblasts, CHO cells, COS cells, NSO cells, HeLa cells, BHK cells, HEK 293 cells or human cells. In a specific embodiment of the present application, the host cell is preferably an immune cell, and the immune cell comprises but is not limited to: T cells, B cells, NK cells, iNKT cells, CTL cells, dendritic cells, myeloid cells, monocytes, macrophages, or any combination thereof, preferably, T cells. In addition, the "host cell" in the present application may comprise a single cell or a cell group, *i.e.,* the "engineered host cell" in the present application comprises a single engineered host cell and an engineered host cell group.

### Immunoconjugate or detection reagent

According to another aspect, the present application provides an immunoconjugate or a reagent for detecting a BCMA protein.

Further, the immunoconjugate comprises the nanobody according to the first aspect of the present application and a conjugate moiety conjugated to the nanobody.

Further, the conjugate moiety comprises a detectable marker, a radionuclide, a cytokine, a therapeutic agent, a cytotoxin, an enzyme, a gold nanoparticle/nanorod, a nanomagnetic particle, a viral coat protein, a virus-like particle (VLP), or a combination thereof.

Further, the detectable marker comprises a fluorescent or luminescent marker, a radioactive label, or a magnetic resonance imaging (MRI) or CT (X-ray computed tomography) contrast agent. Further, the radionuclide comprises ¹³¹I, ³²P, ⁸⁹Sr, ⁹⁰Y, ²²³Ra, ¹²⁵I, or ¹⁰³Pd. Further, the cytokine comprises IL-2, IL-3, IL-4, IL-5, IL-6, IL-9, IL-10, IL-12, IL-13, IL-14, IFN-γ, TNF-β, TNF-α, G-CSF, or M-CSF. Further, the therapeutic agent comprises an alkylating agent, an antimetabolite, an anti-tumor antibiotic, a mitotic inhibitor, a chromatin function inhibitor, an anti-angiogenic agent, an anti-estrogen, an anti-androgen, or an immunomodulator. Further, the alkylating agent comprises bis-chloroethyl methylamine, chlorambucil, phenylalanine mustard, pipobroman, prednimustine, estramustine phosphate, cyclophosphamide, hexamethylmelamine, ifosfamide, fortepidine, thiotepa, carmustine, streptozotocin, improsulfan, dacarbazine, cisplatin, oxaliplatin, or carboplatin. Further, the antimetabolites comprise methotrexate, 5-fluorouracil, floxuridine, 5-fluorodeoxyuracil, capecitabine, cytarabine, fludarabine, 6-mercaptopurine, 2-chlorodeoxyadenosine, 5-azacytidine, 2,2-difluorodeoxycytidine, cladribine, deoxycoformycin, or pentostatin. Further, the anti-tumor antibiotic comprises daunorubicin, doxorubicin, idarubicin, plicamycin, mitomycin C, valrubicin, mitoxantrone hydrochloride, bleomycin, dactinomycin, mithramycin A, or procarbazine. Further, the mitotic inhibitor comprises docetaxel, vinblastine, paclitaxel, vincristine, vindesine, or vinorelbine. Further, the chromatin function inhibitor comprises irinotecan, etoposide, topotecan, etoposide phosphate, or teniposide. Further, the anti-angiogenic agent comprises prinomastat, tanomastat, ilomastat, razoxane, marimastat, batimastat, CGS-27023A, halofuginone, COL-3, neovastat, BMS-275291, and thalidomide. Further, the anti-estrogen comprises toremifene, raloxifene, tamoxifen, anastrozole, letrozole, droloxifene, odoxifene, or exemestane. Further, the anti-androgen comprises nilutamide, bicalutamide, spironolactone, flutamide, finasteride, cyproterone acetate, or cimetidine. Further, the immunomodulator comprises interleukin, tumor necrosis factor, interferon, mushroom polysaccharide, sizofiran, roquinimex, pidotimod, methoxypolyethylene glycol succinamide adenosine deaminase, or a thymosin preparation. Further, the cytotoxin comprises MMAE, DM1, Ozogamicin, Dxd, SN-38, MMAF, PBD, DM2, Amanitin, DM4, PNU-159682, IR700, PE-38, PE24, PE-T20, PE-T20-KDEL, PE4E, or PE40.

Further, the reagent for detecting a BCMA protein comprises the nanobody according to the first aspect of the present application or the immunoconjugate.

Further, the reagent further comprises a diagnostic agent coupled to the nanobody or the immunoconjugate.

Further, the diagnostic agents comprise a radionuclide, a chemiluminescent agent, a bioluminescent agent, a paramagnetic ion, an enzyme, or a photosensitive diagnostic agent. Further, the radionuclide comprises ¹⁸F , ⁵²Fe , ⁶²Cu, ⁶⁴Cu, ⁶⁷Cu, ⁸⁶Y, ⁹⁰Y, ⁸⁹Zr, ¹²⁰I, ¹²³I, ¹²⁴I, ¹²⁵I, ¹³¹I, ¹³N, ¹⁵O, ¹⁸⁶Re, ¹⁸⁸Re, ⁵¹Mn, ⁵⁵Co, or ⁷²As. Further, the chemiluminescent agent comprises luminol, isoluminol, an aromatic acridinium ester, an imidazole, an acridinium salt, or an oxalate ester. Further, the bioluminescent agent comprises fluorescein, luciferase, or aequorin. Further, the paramagnetic ion comprises chromium (III), manganese (II), iron (III), iron (II), cobalt (II), nickel (II), copper (II), neodymium (III), samarium (III), ytterbium (III), gadolinium (III), vanadium (II), terbium (III), dysprosium (III), holmium (III), or erbium (III). Further, the enzyme comprises horseradish peroxidase, alkaline phosphatase, glucose oxidase, β-D-galactosidase, urease, catalase, or glucoamylase. Further, the photosensitive diagnostic agent comprises dihydroxysilicon phthalocyanine, methylene blue, protoporphyrin, hematoporphyrin, or photofrin.

### Pharmaceutical composition or kit

According to another aspect, the present application provides a pharmaceutical composition or a kit.

Further, the pharmaceutical composition comprises the nanobody according to the first aspect of the present application, the engineered host cell according to the sixth aspect of the present application, and/or the immunoconjugate according to the seventh aspect of the present application.

Further, the pharmaceutical composition further comprises one or more pharmaceutically or physiologically acceptable carriers and/or excipients. Further, the pharmaceutical composition is used for treating and/or preventing a BCMA-positive related disease.

Further, the BCMA-positive related disease comprises multiple myeloma, acute myeloid leukemia, chronic myeloid leukemia, chronic lymphocytic leukemia, acute lymphocytic leukemia, diffuse large B-cell lymphoma, Hodgkin's lymphoma, non-Hodgkin's lymphoma, gastric cancer, liver cancer, kidney cancer, lung cancer, small intestine cancer, bone cancer, prostate cancer, colorectal cancer, breast cancer, bowel cancer, cervical cancer, ovarian cancer, lymphoma, nasopharyngeal carcinoma, adrenal tumor, bladder tumor, non-small cell lung cancer, brain glioma, endometrial cancer, fibrosarcoma, myxosarcoma, liposarcoma mesothelioma, lymphatic malignancies, or pancreatic cancer.

Further, the kit comprises the nanobody according to the first aspect of the present application and the immunoconjugate or the reagent according to the seventh aspect of the present application.

Further, the kit is used for detecting a BCMA protein.

In some embodiments, the kit further comprises a container, instructions for use, a buffer, *etc.* In some other embodiments, the kit further comprises a lysis medium for dissolving a sample to be tested, general reagents and buffers required for detection, such as various buffers, detection markers, detection substrates, *etc.* The detection reagent kit may be an in-vitro diagnostic device.

In some embodiments, the pharmaceutical composition of the present application may also comprise other therapeutic agents that can be used for adjuvant treatment and/or prevention of a BCMA-positive related disease. The nanobody according to the first aspect of the present application, the engineered host cell according to the sixth aspect of the present application, the immunoconjugate according to the seventh aspect of the present application, and/or other therapeutic agents in the pharmaceutical composition may be administered simultaneously, separately, or sequentially.

In some embodiments, the pharmaceutically or physiologically acceptable carrier and/or excipient may comprise a sterile injectable liquid (such as an aqueous or non-aqueous suspension or solution). In some exemplary embodiments, such sterile injectable liquid is selected from water for injection (WFI), bacteriostatic water for injection (BWFI), sodium chloride solution (*e.g.,* 0.9% (w/v) NaCl), glucose solution (*e.g.,* 5% glucose), a solution containing a surfactant (*e.g.,* 0.01% polysorbate 20), a pH buffer solution (*e.g.,* phosphate buffer solution), Ringer's solution, and any combination thereof.

In some embodiments, the nanobody according to the first aspect of the present application, the engineered host cell according to the sixth aspect of the present application, and/or the immunoconjugate according to the seventh aspect of the present application comprised in the pharmaceutical composition is a "therapeutically effective amount" or "prophylactically effective amount" of the nanobody according to the first aspect of the present application, the engineered host cell according to the sixth aspect of the present application, and/or the immunoconjugate according to the seventh aspect of the present application. The "prophylactically effective amount" refers to an amount sufficient to prevent, hinder or delay the occurrence of a disease. The "therapeutically effective amount" refers to an amount sufficient to cure or at least ameliorate a disease and its complications in a patient who already has the disease. The therapeutically effective amount may vary according to the following factors: the severity of the disease to be treated, the overall state of the immune system of the patient, the general condition of the patient such as age, weight, and gender, the mode of administration of the pharmaceutical composition, other treatment methods used at the same time, *etc.*

In some embodiments, the dose and frequency (single or multiple doses) of the pharmaceutical composition administered to the subject may vary according to a variety of factors, such as whether the mammal suffers from another disease and the route of administration, the age, gender, health status, weight, body mass index, and diet of the subject, the nature and extent of symptoms of the disease being treated (such as cancer symptoms and the severity of such symptoms), the types of treatment methods used at the same time, complications caused by the disease being treated or other health-related problems, *etc.* Other treatment regimens or agents may be used in combination with the pharmaceutical composition and treatment method of the present application. Adjustment and control of the established dose (*e.g.,* frequency and duration) are well within the capabilities of those skilled in the art.

In some embodiments, the pharmaceutical composition may have any one of the following dosage forms: tablets, pills, powder, granules, capsules, suspensions, solutions, emulsions, syrups, sterile aqueous solutions, non-aqueous solutions, suspensions, emulsions, lyophilized preparations, and suppositories. In addition, the pharmaceutical composition can be administered once or multiple times. In this case, the pharmaceutical composition is administered in the form of a liquid preparation, powder, aerosol, capsule, vaginal tablet, capsule, or suppository. The route of administration may comprise, but is not limited to: intraperitoneal, intravenous, intramuscular, subcutaneous, intradermal, oral, topical, intranasal, intrapulmonary, intrarectal, *etc.* When administered orally, the pharmaceutical composition may comprise a coating that protects the active ingredient in the pharmaceutical composition to prevent degradation of the active ingredient in the stomach. In addition, the active ingredient may be administered by any device that can be transferred to the target cell. In a specific embodiment, the pharmaceutical composition provided by the present application may be prepared into various dosage forms according to actual needs, and may be administered in a dosage that is beneficial to the patient by a clinician according to factors such as the type, age, weight, and general disease condition of the subject, and the mode of administration. The mode of administration may be, for example, injection or any other suitable administration mode known to those skilled in the art.

In some embodiments, the pharmaceutical composition, the nanobody, the engineered host cell, the immunoconjugate, *etc.* provided by the present application may be used in combination with other therapies, treatments, or agents. The other therapies, treatments, or agents comprise, but are not limited to: chemotherapy, radiotherapy, surgery, transplantation, adoptive cell therapy, antibodies, cytotoxic agents, chemotherapeutic agents, cytokines, growth inhibitors, antihormonal agents, kinase inhibitors, antiangiogenic agents, cardioprotective drugs, immunostimulants, immunosuppressants, immune checkpoint inhibitors, antibiotics, angiogenesis inhibitors, metabolic regulators or other therapeutic agents, or any combination thereof. In some embodiments, the other agents comprise a protein, peptide, nucleic acid, small molecule agent, cell, toxin, lipid, carbohydrate, or a combination thereof, or any other type of therapeutic agent, such as radiation. In some embodiments, the other therapies, treatments, or agents comprise surgery, chemotherapy, radiation therapy, transplantation, administration of cells expressing recombinant receptors (*e.g.,* chimeric antigen receptors), kinase inhibitors, immune checkpoint inhibitors, mTOR pathway inhibitors, immunosuppressants, immunomodulators, antibodies, immunoablative agents, antibodies and/or antigen-binding fragments thereof, antibody conjugates, other antibody therapies, cytotoxins, steroids, cytokines, peptide vaccines, hormone therapy, antimetabolites, metabolic regulators, drugs that inhibit the calcium-dependent phosphatase calcineurin or p70S6 kinase FK506 or inhibit p70S6 kinase, alkylating agents, anthracyclines, vinca alkaloids, proteasome inhibitors, GITR agonists, protein tyrosine phosphatase inhibitors, protein kinase inhibitors, oncolytic viruses, and/or other types of immunotherapy. In some embodiments, the other agents or treatments comprise bone marrow transplantation, T cell ablative therapy using a chemotherapeutic agent (such as fludarabine), external beam radiation therapy (XRT), cyclophosphamide, and/or antibody therapy.

### Production method, preparation method, detection method, and diagnostic method

According to another aspect, the present application provides any one of the following methods.
(1) A method for producing the nanobody according to the first aspect of the present application, comprising the following steps:
   (a) culturing the engineered host cell according to the sixth aspect of the present application under conditions suitable for the production of the nanobody, to obtain a culture containing the nanobody;
   (b) isolating or recovering the nanobody from the culture obtained in the step (a); and
   (c) purifying the nanobody obtained in the step (b) to obtain the nanobody according to the first aspect of the present application.
(2) A method for preparing the engineered host cell according to the sixth aspect of the present application, comprising the following steps: introducing the nucleic acid molecule according to the fourth aspect of the present application or the expression vector according to the fifth aspect of the present application into a host cell.
   Further, methods of the introduction comprise lipid transfection, microinjection, electroporation, DNA vector, RNA vector, retroviral vector, lentiviral vector, poxvirus vector, herpes simplex virus vector, adenovirus vector, and adeno-associated virus vector. Further, the host cell is a eukaryotic cell or a prokaryotic cell. Further, the host cell is an immune cell. Further, the immune cell comprises T cells, B cells, NK cells, iNKT cells, CTL cells, dendritic cells, myeloid cells, monocytes, macrophages, gdT cells, any immune cells derived from iPS, or any combination thereof. Further, the immune cell is T cells.
(3) A method for detecting a BCMA protein, comprising the following steps: contacting a sample to be tested with the nanobody according to the first aspect of the present application and/or the immunoconjugate or reagent according to the seventh aspect of the present application to detect presence of an antibody-antigen complex.
(4) A method for diagnosing whether a subject has a BCMA-positive related disease, comprising the following steps: contacting a sample to be tested from the subject with the nanobody according to the first aspect of the present application and/or the immunoconjugate or reagent according to the seventh aspect of the present application to detect formation of a complex between the nanobody, the immunoconjugate, and/or the reagent and BCMA or an amount of the complex, wherein the formation of the complex indicates that BCMA or a cell expressing BCMA is present, and the subject has a BCMA-positive related disease.

In some embodiments, the method for diagnosing whether a subject has a BCMA-positive related disease may further comprise: a step of comparing the amount of the BCMA in the sample from the subject with a reference value. The reference value may be the level of BCMA in a sample from a subject (*e.g.,* a healthy control) known to have no disease associated with BCMA (also referred to as a "negative reference value"). For example, if the amount of the BCMA in the sample from the subject is greater than the negative reference value, it indicates that the subject has a disease associated with BCMA.

In the present application, the term "sample to be tested" refers to a collection (such as fluid, cell, or tissue) separated from a subject, as well as fluid, cell, or tissue present in the subject. Examples of the sample to be tested comprise biological fluids, such as blood, serum and serosal fluid, plasma, lymph, urine, saliva, cystic fluid, tears, excretions, sputum, mucosal secretions of secretory tissues or secretory organs, vaginal secretions, ascites, fluids in the pleural, pericardial, peritoneal, abdominal and other body cavities, fluids collected by bronchial lavage fluid, synovial fluid, liquid solutions in contact with a subject or biological source, such as culture medium (comprising conditioned medium), lavage fluid, *etc.,* tissue biopsy samples, fine needle aspirated tissues, surgically removed tissues, organ cultures, or cell cultures.

In the present application, the term "subject" comprises humans and non-human animals. Non-human animals comprise all vertebrates (such as mammals and non-mammals) such as non-human primates (*e.g.,* cynomolgus monkeys), sheep, dogs, cows, chickens, amphibians, and reptiles. In some embodiments, the "subject" is preferably a human.

(5) A method for treating a BCMA-positive related disease, comprising the following steps: administering an effective amount of the nanobody according to the first aspect of the present application, the engineered host cell according to the sixth aspect of the present application, the immunoconjugate according to the seventh aspect of the present application, and/or the pharmaceutical composition according to the eighth aspect of the present application to a subject in need thereof.

In some embodiments, routes of the administration comprise parenteral, intrapulmonary, or intranasal administration, and if local treatment is desired, intralesional administration. Parenteral infusions comprise intramuscular, intravenous, intraarterial, intraperitoneal, or subcutaneous administration. Administration can be by any appropriate route, for example, by injection, such as intravenous or subcutaneous injection, depending in part on whether the administration is short-term or chronic. A variety of administration-time regimens are contemplated herein, comprising but not limited to: single administration or multiple administrations at multiple time points, bolus administration, and pulse infusions.

### Use in the manufacture of therapeutic reagents and diagnostic reagents, and in diagnosis and treatment

According to another aspect, the present application provides use of any one of the following aspects:
(1) use of the nanobody according to the first aspect of the present application for detecting a BCMA protein and/or diagnosing a BCMA-positive related disease;
(2) use of the nanobody according to the first aspect of the present application in the manufacture of a reagent or kit for detecting a BCMA protein and/or diagnosing a BCMA-positive related disease;
(3) use of the reagent according to the seventh aspect of the present application or the kit according to the eighth aspect of the present application for detecting a BCMA protein and/or diagnosing a BCMA-positive related disease;
(4) use of the nanobody according to the first aspect of the present application, the chimeric antigen receptor according to the second aspect of the present application, the chimeric antigen receptor according to the third aspect of the present application, the nucleic acid molecule according to the fourth aspect of the present application, or the expression vector according to the fifth aspect of the present application in preparation of an engineered host cell for treatment and/or prevention of a BCMA positive-associated disease;
(5) use of the nanobody according to the first aspect of the present application, the chimeric antigen receptor according to the second aspect of the present application, the chimeric antigen receptor according to the third aspect of the present application, the nucleic acid molecule according to the fourth aspect of the present application, the expression vector according to the fifth aspect of the present application, or the engineered host cell according to the sixth aspect of the present application in preparation of an immunoconjugate for treatment and/or prevention of a BCMA positive-associated disease;
(6) use of the nanobody according to the first aspect of the present application, the chimeric antigen receptor according to the second aspect of the present application, the chimeric antigen receptor according to the third aspect of the present application, the nucleic acid molecule according to the fourth aspect of the present application, the expression vector according to the fifth aspect of the present application, the engineered host cell according to the sixth aspect of the present application, or the immunoconjugate according to the seventh aspect of the present application in preparation of a medicament for treatment and/or prevention of a BCMA-positive related disease;
(7) use of the nanobody according to the first aspect of the present application, the chimeric antigen receptor according to the second aspect of the present application, the chimeric antigen receptor according to the third aspect of the present application, the nucleic acid molecule according to the fourth aspect of the present application, the expression vector according to the fifth aspect of the present application, the engineered host cell according to the sixth aspect of the present application, the immunoconjugate according to the seventh aspect of the present application, or the pharmaceutical composition according to the eighth aspect of the present application in preparation of a biological agent for treatment and/or prevention of a BCMA positive-associated disease; and
(8) use of the nanobody according to the first aspect of the present application, the chimeric antigen receptor according to the second aspect of the present application, the chimeric antigen receptor according to the third aspect of the present application, the nucleic acid molecule according to the fourth aspect of the present application, the expression vector according to the fifth aspect of the present application, the engineered host cell according to the sixth aspect of the present application, the immunoconjugate according to the seventh aspect of the present application, or the pharmaceutical composition according to the eighth aspect of the present application in preparation of a biological agent for treatment and/or prevention of a BCMA positive-associated disease.

Further, the BCMA-positive related disease comprises multiple myeloma, acute myeloid leukemia, chronic myeloid leukemia, chronic lymphocytic leukemia, acute lymphocytic leukemia, diffuse large B-cell lymphoma, Hodgkin's lymphoma, non-Hodgkin's lymphoma, gastric cancer, liver cancer, kidney cancer, lung cancer, small intestine cancer, bone cancer, prostate cancer, colorectal cancer, breast cancer, colon cancer, cervical cancer, ovarian cancer, lymphoma, nasopharyngeal carcinoma, adrenal tumor, bladder tumor, non-small cell lung cancer, brain glioma, endometrial cancer, fibrosarcoma, myxosarcoma, liposarcoma mesothelioma, lymphatic malignancies, and pancreatic cancer.

In the present application, the term "BCMA-positive related disease" refers to any disease or disorder associated with the expression of BCMA, comprising but not limited to: multiple myeloma, acute myeloid leukemia, chronic myeloid leukemia, chronic lymphocytic leukemia, acute lymphocytic leukemia, diffuse large B-cell lymphoma, Hodgkin's lymphoma, non-Hodgkin's lymphoma, gastric cancer, liver cancer, kidney cancer, lung cancer, small intestine cancer, bone cancer, prostate cancer, colorectal cancer, breast cancer, colon cancer, cervical cancer, ovarian cancer, lymphoma, nasopharyngeal carcinoma, adrenal tumor, bladder tumor, non-small cell lung cancer, brain glioma, endometrial cancer, fibrosarcoma, myxosarcoma, liposarcoma mesothelioma, lymphatic malignancies, and pancreatic cancer. Any disease or disorder associated with the expression of BCMA falls within the scope of protection of the present application.

In the present application, the term "treatment" refers to the complete or partial amelioration or alleviation of a disease or condition or disorder, or symptoms, adverse effects or consequences, or phenotypes associated therewith. Desired therapeutic effects comprise, but are not limited to: preventing the occurrence or recurrence of a disease, alleviating symptoms, reducing any direct or indirect pathological consequences of a disease, preventing cancer metastasis, slowing disease progression, improving or alleviating the disease condition, and alleviating or improving prognosis. The term does not imply a complete cure of the disease or complete elimination of any symptoms or an effect on all symptoms or consequences.

In the present application, the term "delaying disease progression" refers to delaying, hindering, slowing, stopping, stabilizing, suppressing, and/or postponing the development of a disease (such as cancer). Depending on the disease being treated and/or the medical history of the subject, this delay may be of varying lengths of time. A sufficient or significant delay may actually encompass prevention, so that the individual does not develop the disease. For example, advanced cancers, such as cancer metastasis development, may be delayed.

In the present application, the term "prevention" comprises providing a preventive effect in terms of the occurrence or recurrence of a disease in a subject who may be susceptible to the disease but has not yet been diagnosed with the disease. In some embodiments, the molecule and composition provided are used to delay disease development or slow disease progression.

In the present application, the term "suppressing" a function or activity is to reduce a function or activity when compared to otherwise identical conditions except for the condition or parameter of interest or compared to another condition. For example, an antibody or composition or cell that suppresses tumor growth reduces the tumor growth rate compared to the tumor growth rate in the absence of the antibody or composition or cell.

Compared with the prior art, the advantages and beneficial effects of the present application are as follows.
(1) The present application provides nanobodies VHH01 and VHH02 targeting BCMA. Amino acid sequences of CDR1, CDR2, and CDR3 of the nanobody VHH01 are as shown in SEQ ID NO: 3, SEQ ID NO: 5, and SEQ ID NO: 7, respectively. Amino acid sequences of CDR1, CDR2, and CDR3 of the nanobody VHH02 are as shown in SEQ ID NO: 11, SEQ ID NO: 13, and SEQ ID NO: 15, respectively. The nanobodies have strong specificity and high affinity for BCMA, can specifically bind to BCMA-positive cell lines with high affinity, and have the advantages such as good stability, simple structure, and being easy to be engineered, *etc.,* which provides a new idea for the development of effective immunotherapeutic drugs targeting BCMA.
(2) The present application provides a chimeric antigen receptor comprising nanobodies VHH01 and/or VHH02, and also provides BCMA-targeting CAR-T cells prepared based on the chimeric antigen receptor. The CAR-T cells can specifically recognize BCMA-positive tumor cell lines, have high specific killing efficiency on BCMA-positive cell lines, and have no adverse effects on cell lines that do not express BCMA, thereby avoiding the therapeutic toxicity and safety issues caused by off-target effects. This provides a potential treatment option for diseases related to BCMA expression in the art, and has important application prospects and clinical value.

### BRIEF DESCRIPTION OF DRAWINGS

FIG. 1 is a schematic diagram of an alpaca immunization and antibody screening process;
FIG. 2 shows the statistical result of nanobody library enrichment screening;
FIG. 3 shows the statistical result of anti-BCMA nanobody monoclonal screening;
FIG. 4 shows the results of flow cytometry assay of the overexpression cell line K562-BCMA;
FIG. 5 is a schematic diagram of a single VHH CAR structure, wherein section A is a schematic composition diagram of the single VHH CAR structure, and section B is a schematic diagram of the single VHH CAR structure displayed on the cell surface;
FIG. 6 is a representative diagram of the results of single VHH CAR-T cell transduction rate determination;
FIG. 7 shows the statistical result of MFI values on the surface of single VHH CAR-T cells;
FIG. 8 shows an expansion curve of single VHH CAR-T cells;
FIG. 9 shows the statistical result of the CD4/CD8 ratio during single VHH CAR-T culture;
FIG. 10 is the results of a killing experiment of single VHH CAR-T cells against K562-BCMA;
FIG. 11 is a schematic diagram of a dual VHH CAR structure, wherein section A is a schematic composition diagram of the dual VHH CAR structure, and section B is a schematic diagram of the dual VHH CAR structure displayed on the cell surface;
FIG. 12 shows the statistical result of MFI values on the surface of K562-dNMC003-A & K562-dNMC003-B cells;
FIG. 13 is a representative diagram of the results of dNMC003-A & dNMC003-B CAR-T cell transduction rate determination;
FIG. 14 shows an expansion curve of dNMC003-A & dNMC003-B CAR-T cells;
FIG. 15 shows the statistical result of the viability during dNMC003-A & dNMC003-B CAR-T cell culture;
FIG. 16 shows the statistical result of the CD4/CD8 ratio during dNMC003-A & dNMC003-B CAR-T culture;
FIG. 17 shows the results of in vitro functional verification of dNMC003-A & dNMC003-B CAR-T, wherein section A shows the killing results of dNMC003-A & dNMC003-B and NMC003-01 & NMC003-02 on cell lines K562-BCMA and K562, and section B shows the killing results of dNMC003-A & dNMC003-B on cell lines RPMI 8226 and U87-MG;
FIG. 18 shows the results of IFN-γ detection after co-incubation of dNMC003-A & dNMC003-B CAR-T cells with K562-BCMA, RPMI8226, and U87-MG cells;
FIG. 19 shows the affinity assay results of Nb003-01, Nb003-02, dNb003-A, and dNb003-B (by ELISA), wherein section A shows the affinity assay results of Nb003-01 and human BCMA antigen, section B shows the affinity assay results of Nb003-02 and human BCMA antigen, section C shows the affinity assay results of dNb003-A and human BCMA antigen, and section D shows the affinity assay results of dNb003-B and human BCMA antigen;
FIG. 20 shows the affinity assay results of Nb003-01, Nb003-02, and dNb003-B (by SPR), wherein section A shows the affinity assay results of Nb003-01 and human BCMA antigen, section B shows the affinity assay results of Nb003-02 and human BCMA antigen, and section C shows the affinity assay results of dNb003-B and human BCMA antigen; and
FIG. 21 shows the specificity verification results of Nb003-01, Nb003-02, dNb003-A, and dNb003-B.

### DETAILED DESCRIPTION

The present application is further described below in conjunction with specific embodiments, which are merely used to explain the present application and should not be construed as a limitation to the present application. Those having ordinary skills in the art should understand that various changes, modifications, replacements and variations may be made to the embodiments without departing from the principles and spirit of the present application, and the scope of the present application is as defined by the appended claims and their equivalents. The methods given in examples below are all conventional methods, unless otherwise stated. The methods given in examples below are all conventional methods, unless otherwise stated. The reagents and materials used in examples below are all commercially available, unless otherwise stated.

### Example 1 Screening of anti-BCMA nanobodies

### 1. Animal immunization and titer determination

### (1) Preparation of antigen

The RNA in RPMI 8226 cells was extracted using an RNA extraction kit. Referring to the instruction manual of SuperScript^{™} II Reverse Transcriptase, random primers were used for reverse transcription to obtain cDNA. Using cDNA as a template, the extracellular region gene sequence of the antigen BCMA was obtained by PCR. The extracellular region gene sequence of BCMA was ligated into a protein expression vector for expression, and Ni column purification was performed to obtain purified BCMA protein.

### (2) Animal immunization

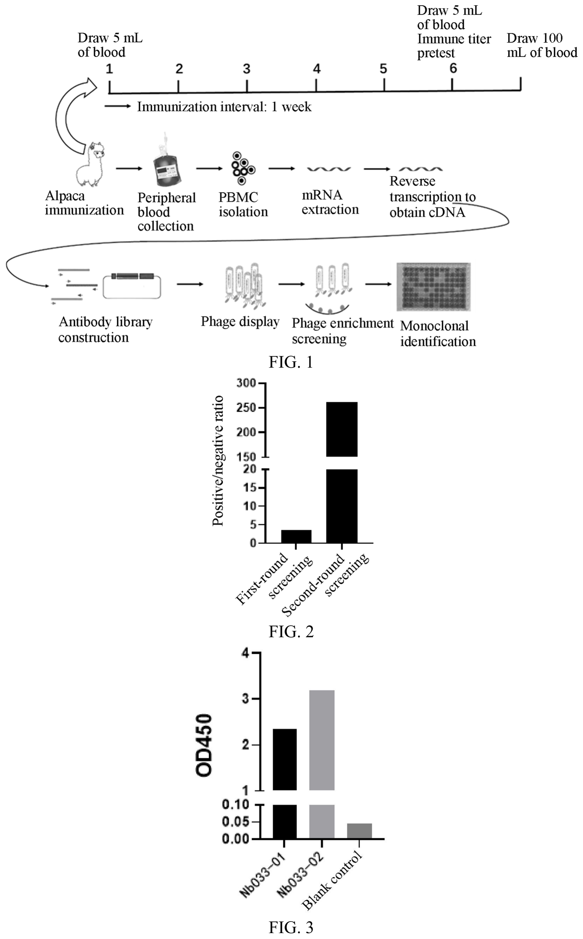

Alpaca immunization was performed using the BCMA-His protein purified by the present application. The specific alpaca immunization process is shown in FIG. 1. Immunization was performed once a week, and a total of 6 consecutive immunizations were performed. Before the 6th immunization, 5 mL of blood was drawn for titer pretesting, and 100 mL of peripheral blood was collected 7 days after the last immunization for the construction and screening of the antibody library.

### 2. Construction and enrichment screening of nanobody library

### (1) Construction of nanobody library

Seven days after the last immunization, 100 mL of peripheral blood was collected from alpacas, peripheral blood mononuclear cells were separated by Ficoll density gradient centrifugation, and RNA was extracted. The cDNA was prepared using a reverse transcription kit.

Using cDNA as a template, the immunoglobulin heavy chain region-specific primers CALL001 and CALL002 were used to amplify the variable regions of all immunoglobulin heavy chains (VHs and VHH) from cDNA. The fragment of about 700 bp represents the antibody library with only heavy chains, and the fragment of about 1000 bp corresponds to the heavy chain of traditional antibodies. The amplification product was analyzed using 1% (wt/Vol) agarose gel. The product of about 700 bp was recovered by gel recovery.

Using the gel recovery product as a template, the VHHs sequence was specifically amplified using degenerate primers VHH-BACK and VHH-FOR, and the amplification product was analyzed using 1% (wt/Vol) agarose gel. It was ligated into the pMES4 phage display vector, and the resultant product was electrotransformed into the electrotransformation competent cell TG1. The resulting bacterial library was the constructed BCMA single domain heavy chain antibody phage display library. After the library was constructed, to detect the insertion efficiency of the library, 25 clones were randomly selected for colony PCR using primers MP57 and GIII, and the PCR products were subjected to Sanger sequencing. The construction process of the nanobody library is shown in FIG. 1. The primer sequences used are shown in Table 1.

**Table 1 List of primers**

| Primer name | Primer sequence |
|---|---|
| CALL001 | 5'-GTCCTGGCTGCTCTTCTACAAGG-3' |
| CALL002 | 5'-GGTACGTGCTGTTGAACTGTTCC-3' |
| VHH-BACK | 5'-GATGTGCAGCTGCAGGAGTCTGGRGGAGG-3' |
| VHH-FOR | 5'-CTAGTGCGGCCGCTGGAGACGGTGACCTGGGT-3' |
| MP57 | 5'-TTATGCTTCCGGCTCGTATG-3' |
| GIII | 5'-CCACAGACAGCCCTCATAG-3' |

### (2) Enrichment screening of antibody library

The TG1 Escherichia *coli* nanobody library was transferred to 2-YT liquid culture medium and cultured at 37°C and 200 rpm until the OD value reached 0.5. Then, the helper phage VCSM13 was added to infect the cells. After gentle mixing, the cells were incubated at 37°C for 30 minutes. The bacterial liquid was centrifuged to remove trace glucose, and the pellet was resuspended in 2-YT culture medium with ampicillin and kanamycin resistance, and cultured overnight at 37°C and 200 rpm to amplify the phages displaying nanobodies. The overnight culture was transferred to a 50 mL centrifuge tube, the supernatant was centrifuged, and 20% (wt/vol) PEG6000/2.5 M NaCl solution was added to precipitate the phages. The supernatant was discarded by centrifugation and the pellet was resuspended in 1 mL of PBS. After centrifugation, the supernatant was transferred to a new centrifuge tube, glycerol was added to a final concentration of 20%, and the mixture was stored at -80°C. For titer determination of phage nanobody library, the phages were diluted in a 10-fold gradient, phages of different dilution multiples were taken to infect TG1 bacteria in the logarithmic growth phase, which were then cultured at 37°C overnight. The titer of the phage nanobody library was calculated according to the number of plaques on the second day.

Nanobodies were panned by ELISA, and the recombinant BCMA-His protein was coated on an ELISA plate and incubated overnight at 4°C. The ELISA plate was washed three times with 250 µL of PBST, 200 µL of blocking solution was added, and the ELISA plate was incubated at room temperature for 2 h. The corresponding phage was added to each well and incubated at room temperature for 2 h. The plate was washed 15 times with 250 µL of PBST. 100 µL of trypsin was added to each well and incubated at room temperature at 700 rpm for 0.5 h. The phages were eluted with AEBSF. The eluted phages were titered and amplified by phage infection. When the ratio of the number of phages displaying the nanobody to the number of blank phages among the eluted phages was greater than or equal to 100, the panning was stopped. The enrichment screening process of the antibody library is shown in FIG. 1.

### 3. Monoclonal identification

Single clones were selected from the TG1 *Escherichia coli* library obtained after two rounds of screening for expansion culture, and the helper phage VCSM13 was used for infection to prepare monoclonal phages. The monoclonal phage was added to the ELISA plate coated with BCMA protein blocked with 2% skim milk powder, and incubated at room temperature for 2 h. After the plate was washed with PBST, HA-HRP antibody was added and the plate was incubated at room temperature for 1 h. After the plate was washed with PBST, 100 µL of TMB single-component colorimetric solution was added, and 100 µL of stop solution was added after incubation at room temperature for 30 min. The absorbance at 450 nm was detected by an ELISA instrument. When the ratio of the OD450 value of the sample well to the blank control was greater than 2, it was determined to be a positive clone. The monoclonal identification process is shown in FIG. 1. The positive clones were subjected to bacterial liquid PCR and Sanger sequencing. Sequence alignment was carried out on the monoclones sequenced by Sanger using the software DNAMAN. Sequence-specific clones were screened out.

### 4. Experimental results

The results of the construction of the nanobody library showed that the BCMA nanobody library was successfully constructed, with a library capacity of 3E8 and an insertion rate of nearly 95%.

The panning results of the antibody library are shown in FIG. 2. After two rounds of enrichment screening, the final ratio of the number of phages displaying the nanobody to the number of blank phages reached 262.1, which has reached the criterion for screening monoclonal clones. Therefore, after two rounds of panning, the panning was stopped and the subsequent step of monoclonal screening and identification was carried out.

After sequence identification, two clones with specific sequences and high OD450 values detected by ELISA were finally obtained, which were named Nb003-01 (VHH01) and Nb003-02 (VHH02). The OD450 results are shown in FIG. 3. Amino acid sequences of CDR1, CDR2, and CDR3 of the nanobody VHH01 are as shown in SEQ ID NO: 3, SEQ ID NO: 5, and SEQ ID NO: 7, respectively. Nucleotide sequences of CDR1, CDR2, and CDR3 of the nanobody VHH01 are respectively as shown in SEQ ID NO: 4, SEQ ID NO: 6, and SEQ ID NO: 8. An amino acid sequence of the nanobody VHH01 is as shown in SEQ ID NO: 1. A nucleotide sequence of the nanobody VHH01 is as shown in SEQ ID NO: 2. Amino acid sequences of CDR1, CDR2, and CDR3 of the nanobody VHH02 are as shown in SEQ ID NO: 11, SEQ ID NO: 13, and SEQ ID NO: 15, respectively. Nucleotide sequences of CDR1, CDR2, and CDR3 of the nanobody VHH02 are respectively as shown in SEQ ID NO: 12, SEQ ID NO: 14, and SEQ ID NO: 16. An amino acid sequence of the nanobody VHH02 is as shown in SEQ ID NO: 9. A nucleotide sequence of the nanobody VHH02 is as shown in SEQ ID NO: 10.

### Example 2 Construction of K562-BCMA stable cell line

### 1. Experimental method

Using the cDNA obtained in Example 1 as a template, the full-length sequence of BCMA was obtained by PCR, with Xho I and EcoR I cleavage sites on the two ends. The sequence was inserted into the lentiviral vector pLVX-Puro vector by double restriction enzyme digestion and ligation, and the recombinant plasmid was named pLVX-BCMA-Puro. The recombinant plasmid used the CMV promoter and carried the puromycin resistance gene.

The target plasmid pLVX-BCMA-Puro was packaged with the helper plasmid together with the lentiviral vector. Before CAR-T cells were prepared, lentivirus packaging was first performed. The target plasmid and three helper plasmids (pMD2.G, pRSV-REV, and pMDLg/RRE) were co-transfected into 293FT cells under the action of PEI-Pro. The medium was changed after 6 hours of packaging. The lentivirus was harvested after 48 hours of packaging. The harvested lentivirus stock solution was concentrated by ultracentrifugation, and the lentivirus particles were resuspended in DMEM high-glucose medium and aliquoted for use.

### 2. Experimental results

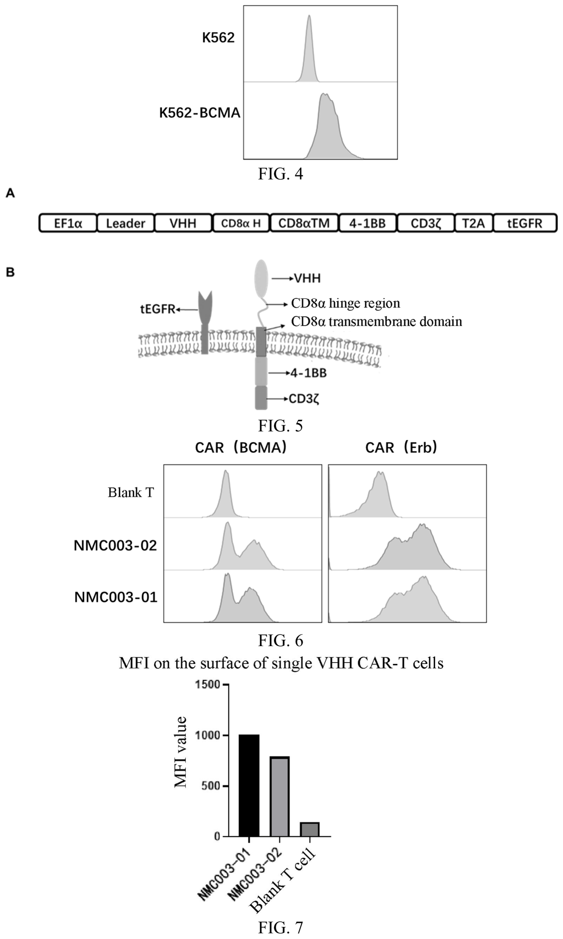

The results are shown in FIG. 4, showing that the constructed K562-BCMA stable cell line highly expresses BCMA, *i.e.,* the present application successfully constructed a K562-BCMA stable cell line.

### Example 3 Preparation and in vitro functional verification of single VHH CAR-T cells

### 1. Construction of single VHH CAR structure

The sequence-specific clone was used to construct a single VHH CAR structure. First, the VHH sequence of the positive clone was amplified with the primers NCAR-F1 and NCAR-R1 using the plasmid that was successfully sequenced as a template. After the first round of PCR, the second round of PCR was performed with the primers NCAR-F2 and NCAR-R2 using the product of the first round of PCR as a template. The product of the second round of PCR was ligated into the vector Senl-S88BZ by homologous recombination, and the vector was digested with Not I. Thus, a CAR structure containing a single VHH targeting BCMA was successfully constructed. FIG. 5 is a schematic diagram of the structure. The primer sequences are shown in Table 2.

**Table 2 Primer list for construction of single VHH CAR structure**

| Primer name | Primer sequence |
|---|---|
| NCAR-F1 | 5'-CTGCAGGAGTCTGGRGGAGG-3' |
| NCAR-R1 | 5'-TGAGGAGACGGTGACCTGGG-3' |
| NCAR-F2 | 5'- TTTCTGCTGATCCCCCAGGTGCAGCTGCAGGAGTCTGGRGGAGG-3' |
| NCAR-R2 | 5'- TAGGAGCCGGGGTGGGCGGCCGCGGTGCTGGGGTAGTTGAGGAG ACGGTGACCTGGG-3' |

A total of two single VHH CAR structures were constructed, named NMC003-01 and NMC003-02, respectively, and the structures are shown in FIG. 5. EF1α is the promoter of elongation factor 1α, Leader is the coding sequence of the signal peptide, VHH is the coding sequence of the anti-BCMA nanobody, CD8αH+TM is the CD8a hinge region and transmembrane region, 4-1BB and CD3ζ intracellular signal regions are intracellular costimulatory domains, and the extracellular region of tEGFR is expressed through T2A peptide linkage to detect CAR expression after lentiviral transduction. The NMC003-01 is obtained by connecting EF1α, a signal peptide, VHH01, a CD8a hinge region, a CD8α transmembrane domain, a 4-1BB costimulatory domain, a CD3ζ intracellular signaling domain, T2A, a tEGFR signal peptide, and tEGFR sequentially in tandem. The NMC003-02 is obtained by connecting EF1α, a signal peptide, VHH02, a CD8α hinge region, a CD8a transmembrane domain, a 4-1BB costimulatory domain, a CD3ζ intracellular signaling domain, T2A, a tEGFR signal peptide, and tEGFR sequentially in tandem. A nucleotide sequence of the EF1α is as shown in SEQ ID NO: 17. Amino acid sequences of the signal peptide, the CD8a hinge region, the CD8α transmembrane domain, the 4-1BB costimulatory domain, the CD3ζ intracellular signaling domain, the T2A, the tEGFR signal peptide, and the tEGFR are respectively as shown in SEQ ID NO: 18 to SEQ ID NO: 25. Nucleotide sequences of the signal peptide, the CD8α hinge region, the CD8a transmembrane domain, the 4-1BB costimulatory domain, the CD3ζ intracellular signaling domain, the T2A, the tEGFR signal peptide, and the tEGFR are respectively as shown in SEQ ID NO: 26 to SEQ ID NO: 33.

### 2. Lentiviral packaging

Before CAR-T cells were prepared, lentivirus packaging was first performed. The target plasmid and three helper plasmids (pMD2.G, pRSV-REV, and pMDLg/RRE) were co-transfected into 293FT cells under the action of PEI-Pro. The medium was changed after 6 hours of packaging. The lentivirus was harvested after 48 hours of packaging. The harvested lentivirus stock solution was concentrated by ultracentrifugation, and the lentivirus particles were resuspended in DMEM high-glucose medium and aliquoted for use.

### 3. Preparation of single VHH CAR-T cells

After lentivirus packaging, CAR-T cells were prepared. Peripheral blood mononuclear cells (PBMC) were collected from patients or healthy donors. α/β T cells were sorted by CD3 magnetic beads. The sorted α/β T cells were cultured in TexMACS GMP medium (MACS). Lentivirus transduction was performed after 2 days. CAR-T cells were harvested after 12 to 14 days of culture to obtain BCMA-targeting VHH CAR-T cells (named NMC003-01 and NMC003-02, respectively). Flow cytometry was performed during the culture process to determine the proportion of CAR+ cells, the expression of tEGFR was detected using an anti-EGFR antibody, the expression of nanobodies on the cell surface was detected using BCMA-His protein as the primary antibody and anti-His tag antibody the secondary antibody, and the MFI values of VHH on the surface of CAR-T cells detected by BCMA-His antigen were statistically analyzed. On the 6th, 9th, and 12th days of cell culture, cells were counted for statistics on cell expansion. Samples were taken for flow cytometry on the 6th and 12th days of culture to calculate the CD4/CD8 ratio of CAR-T cells.

### 4. In vitro functional verification of single VHH CAR-T cells

To verify the in vitro biological activity of the anti-BCMA VHH CAR-T cells prepared in this example, an in vitro killing experiment was performed during the culture process. First, target cells were collected, and the overexpression cell line K562-BCMA (K562 is a human chronic myeloid leukemia cell) described in Example 2 was collected, centrifuged at 2000 rpm for 5 min, resuspended in DPBS and counted, and added to a 96-well plate in an amount of 1×10⁵ cells/well. Then, according to different effector-to-target ratios (E:T=0.3:1, 1:1, and 3:1), an appropriate amount of effector cells was added to the target cells, mixed, and incubated for 4 hours, and the cell killing ratio was detected by flow cytometry.

### 5. Experimental results

The single VHH structure was transduced into T cells to prepare CAR-T cells. The representative flow cytometry test results after 6 days of CAR-T cell culture are shown in FIG. 6. In the figure, CAR(Erb) shows the expression of tEGFR on the cell surface, and CAR(BCMA) shows the expression of nanobodies on the cell surface. The results show that both NMC003-01 and NMC003-02 CAR-T cells specifically expressed CAR(Erb) and CAR(BCMA). The MFIs of nanobodies on the surface of CAR-T cells were statistically analyzed. The results are shown in FIG. 7. It can be seen from the results that compared with blank T, the MFIs of nanobodies on the surface of NMC003-01 and NMC003-02 were significantly improved. The above results prove that tEGFR and nanobodies in the single VHH CAR structure were successfully expressed.

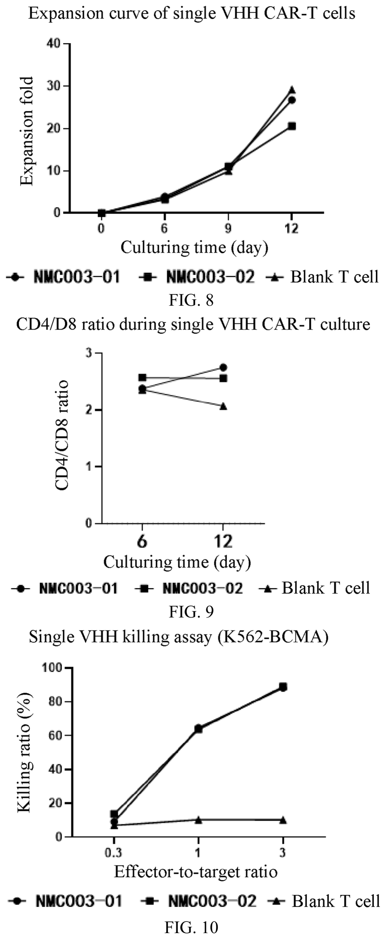

During the culture of single VHH CAR-T cells, the cell expansion fold and the CD4/CD8 ratio were statistically analyzed. FIG. 8 shows an expansion curve of single VHH CAR-T cells. From the beginning of culture to the harvest on day 12, the expansion folds of NMC003-01, NMC003-02, and blank T cells were 26.8, 20.6, and 29.2, respectively. The expansion folds of NMC003-01 and NMC003-02 were close to that of blank T cells. The CD4/CD8 ratio during cell culture is shown in FIG. 9. The CD4/CD8 ratio changed during the culture process. At the time of harvesting, the CD4/CD8 ratios of NMC003-01 and NMC003-02 were 2.75 and 2.56, respectively, and the CD4/CD8 ratio of blank T cells was 2.07. The above results prove that the single VHH CAR-T cells were successfully expanded, and the CD4/CD8 ratio tended to be a normal level during the expansion process.

The in vitro biological activity of single VHH CAR-T cells was verified by a killing experiment against K562-BCMA. FIG. 10 shows the results of an in vitro killing experiment of single VHH CAR-T cells against K562-BCMA. When the killing ratio was 0.3:1, 1:1, and 3:1, NMC003-01 and NMC003-02 both produced specific killing against K562-BCMA. The killing ratio gradually increased with the increase of the effector-to-target ratio. The killing ratios of NMC003-01 and NMC003-02 against K562-BCMA were significantly higher than that of blank T cells. The above results show that single VHH CAR-T cells have produced specific killing of BCMA-positive cell lines.

### Example 4 Preparation and functional verification of dual VHH CAR-T (dNMC003-A, dNMC003-B) cells

### 1. Construction of dNMC003-A and dNMC003-B structures

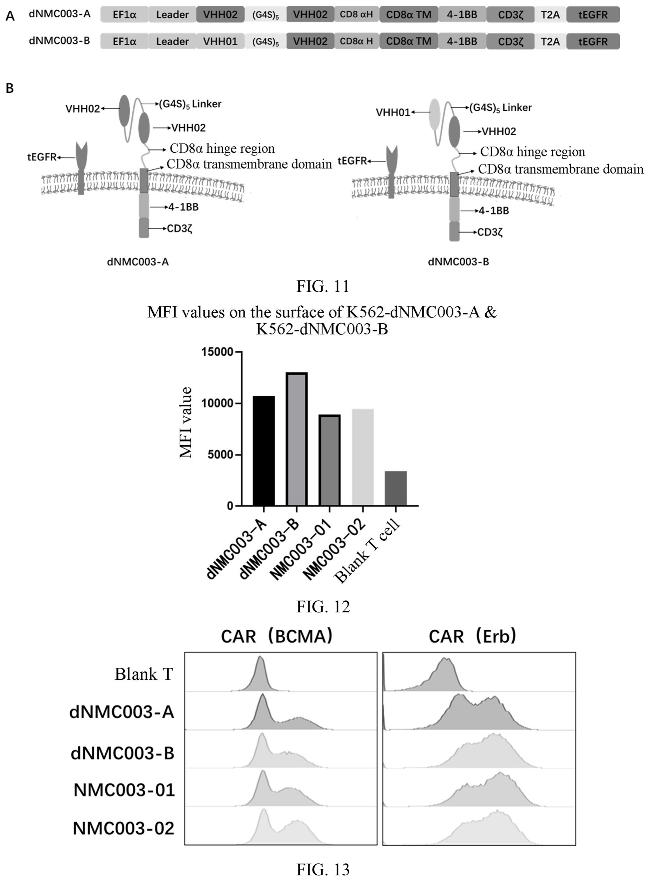

The nanobodies Nb003-01 and Nb003-02 screened by the present application were used to construct a target plasmid of a dual VHH CAR structure. FIG. 11 is a schematic diagram of the structure. The antigen binding region of dNMC003-A comprises two VHH02, and the antigen binding region of dNMC003-B comprises VHH01 and VHH02. First, the VHH02 sequence was amplified by PCR. After the first round of PCR, the second round of PCR was performed with primers using the product of the first round of PCR as a template. The primer sequences are shown in Table 3. Then, the product of the second round of PCR was ligated into vectors NMC003-01 and NMC003-02 by homologous recombination, and the vectors were digested with Not I. The recombinant plasmid ligated into vector NMC003-02 was named dNMC003-A, and the recombinant plasmid ligated into vector NMC003-01 was named dNMC003-B. FIG. 11 is a structural diagram of the structures.

EF1α is the promoter of elongation factor 1α, Leader is the coding sequence of the signal peptide, VHH01 and VHH02 are the coding sequences of the anti-BCMA nanobody, CD8αH+TM is the CD8a hinge region and transmembrane region, 4-1BB and CD3ζ intracellular signal regions are intracellular costimulatory domains, and the extracellular region of tEGFR is expressed through T2A peptide linkage to detect CAR expression after lentiviral transduction. The dNMC003-A is obtained by connecting EF1α, a signal peptide, VHH02, Linker ((G4S)₅), VHH02, a CD8a hinge region, a CD8α transmembrane domain, a 4-1BB costimulatory domain, a CD3ζ intracellular signaling domain, T2A, a tEGFR signal peptide, and tEGFR sequentially. The dNMC003-B is obtained by connecting EF1α, a signal peptide, VHH01, Linker ((G4S)₅), VHH02, a CD8α hinge region, a CD8a transmembrane domain, a 4-1BB costimulatory domain, a CD3ζ intracellular signaling domain, T2A, a tEGFR signal peptide, and tEGFR sequentially. Sequence information of EF1α, the signal peptide, the CD8a hinge region, the CD8a transmembrane domain, the 4-1BB costimulatory domain, the CD3ζ intracellular signaling domain, T2A, the tEGFR signal peptide, and tEGFR is as shown in Example 3.

**Table 3 Primer list for construction of dual VHH CAR structure**

| Primer name | Primer sequence |
|---|---|
| dNCAR-F1 | 5'-CAGGTGCAGCTGCAGGAG-3' |
| dNCAR-R1 | 5'-TGAGGAGACGGTGACCTGG-3' |
| dNCAR-F2 | 5'- CCAGGTCACCGTCTCCTCAGGAGGAGGAGGATCCGGAGGAGGAG GATCTGGCGGCGGCGGCAGTGGCGGCGGCGGCTCCGGCGGCGGC GGCTCTCAGGTGCAGCTGCAGGAG-3' |
| dNCAR-R2 | 5'- TAGGAGCCGGGGTGGGCGGCCGCGGTGCTGGGGTAGTTGAGGAG ACGGTGACCTGG-3' |

### 2. Preparation of K562-dNMC003-A and K562-dNMC003-B cells

Lentivirus packaging was performed according to the preparation process of Example 3. After the lentivirus packaging was completed, the lentivirus was used to transduce the K562 cell line. K562 cells transduced with dNMC003-A and dNMC003-B series lentiviruses were cultured. Flow cytometry was performed after 3 days of culture. The expression of nanobodies on the cell surface was detected using BCMA-His protein as the primary antibody and anti-His tag antibody as the secondary antibody. The MFI of VHH on the surface of K562 was statistically analyzed.

### 3. Preparation of dNMC003-A and dNMC003-B CAR-T cells

According to the preparation process in Example 3, dNMC003-A and dNMC003-B CAR-T cells were cultured, and NMC003-01 and NMC003-02 CAR-T cells were cultured at the same time. Flow cytometry was performed during the culture process to determine the proportion of CAR+ cells, the expression of tEGFR was detected using an anti-EGFR antibody, and the expression of nanobodies on the cell surface was detected using BCMA-His protein as the primary antibody and anti-His tag antibody the secondary antibody. On the 6th, 9th, and 13th days of cell culture, cells were counted for statistics on cell expansion and the proportion of viable cells. Samples were taken for flow cytometry to calculate the CD4/CD8 ratio of CAR-T cells.

### 4. In vitro functional verification of dNMC003-A and dNMC003-B CAR-T cells

To verify the in vitro biological activity of dNMC003-A, dNMC003-B, NMC003-01, and NMC003-02 CAR-T cells prepared in this example, an in vitro killing experiment was performed during the culture process. First, target cells were collected, K562 (human chronic myeloid leukemia cells) and BCMA overexpressing cell line K562-BCMA were respectively collected, centrifuged at 2000 rpm for 5 min, resuspended in DPBS and counted, and added to a 96-well plate in an amount of 1×10⁵ cells/well. Then, according to an effector-to-target ratio (E:T) of 3:1, corresponding effector cells dNMC003-A, dNMC003-B, NMC003-01, and NMC003-02 CAR-T cells were added to the target cells, mixed, and incubated for 4 hours, and the cell killing ratio was detected by flow cytometry.

In addition, the in vitro killing effects of dNMC003-A and dNMC003-B CAR-T cells against human multiple myeloma peripheral blood B lymphocytes RPMI 8226 (purchased from BNCC) and BCMA-negative cell line human brain glioma cell line U87-MG (purchased from BNCC) were tested according to the above method.

In addition, target cells were taken and the cell density was adjusted to 2×10⁶ cells/mL. The target cells were added to a 96-well plate in an amount of 100 µL per well, and the well density was 2×10⁵ cells/well. According to an effector-to-target ratio (E:T) of 3:1, an appropriate amount of effector cells dNMC003-A and dNMC003-B CAR-T cells were added to the target cells in each well, evenly mixed, and incubated together for 18 hours. The supernatant was taken to detect the secretion of IFN-γ.

### 5. Experimental results

After the dual VHH structure was transduced into K562 cells, the MFI statistical results of BCMA antigen staining of K562-dNMC003-A and K562-dNMC003-B cells are shown in FIG. 12. It can be seen from the results that the expression intensity of VHH on the surface of K562-dNMC003-A and K562-dNMC003-B is higher than that on blank T cells, indicating that the nanobodies in the dual VHH CAR structure were successfully expressed in K562 cells.

The representative flow cytometry test results after 6 days of CAR-T cell culture are shown in FIG. 13. In the figure, CAR(Erb) shows the expression of tEGFR on the cell surface, and CAR(BCMA) shows the expression of nanobodies on the cell surface. The results show that both the two dual VHH CAR-T cells dNMC003-A and dNMC003-B specifically expressed CAR(Erb) and CAR(BCMA).

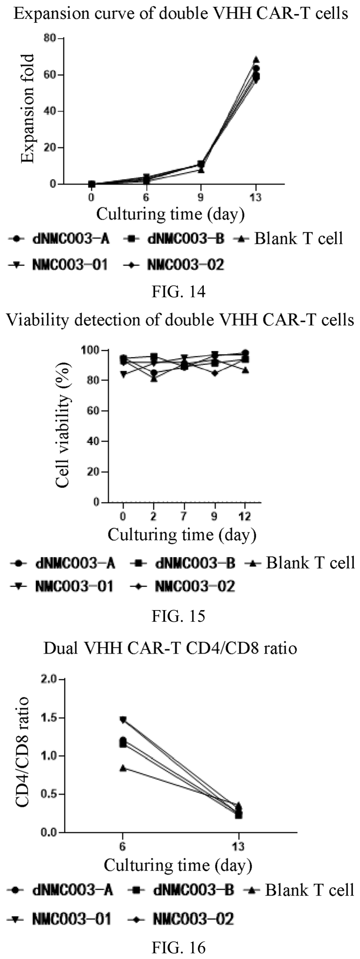

During the culture process of CAR-T cells in this example, the cell expansion fold, CAR-T cell viability, and CD4/CD8 ratio were statistically analyzed. FIG. 14 shows expansion curves of NMC003-01, NMC003-02, dNMC003-A, and dNMC003-B CAR-T. It can be seen from the figure that on the 13th day of expansion, the expansion fold of NMC003-01 was 56.9, the expansion fold of NMC003-02 was 60.6, the expansion fold of dNMC003-A was 63.6, the expansion fold of dNMC003-B was 59.1, and the expansion fold of blank T cells was 68.5. FIG. 15 shows the statistical results of cell viability during the culture of NMC006-04, NMC006-06, and dNMC006-A CAR-T cells. It can be seen from the figure that CAR-T cells maintained a high cell viability during the entire expansion process. The CD4/CD8 ratio during cell culture is shown in FIG. 16. The CD4/CD8 ratio decreased during the culture process. At the time of harvesting, the CD4/CD8 ratios of NMC003-01, NMC003-02, dNMC003-A, and dNMC003-B were 0.24, 0.31, 0.26, and 0.23, respectively, which were close to that of blank T cells (0.36). The above results prove that the dual VHH CAR-T cells were successfully expanded and had a high viability during the expansion process. Compared with blank T cells, the CD4/CD8 ratio tended to be a normal level.

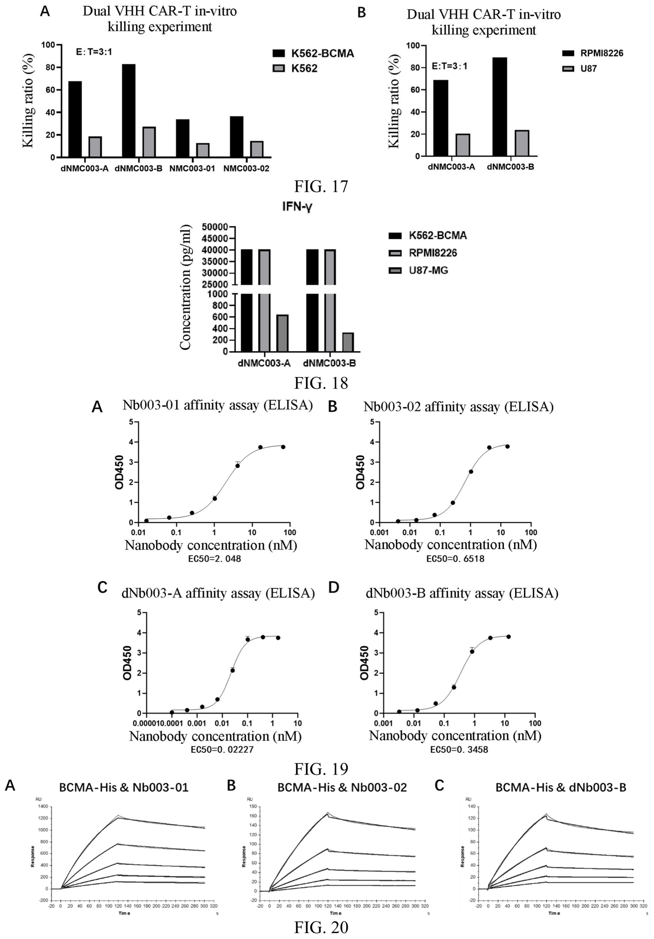

The in vitro biological activity of dual VHH CAR-T cells was verified by killing experiments against K562, K562-BCMA, RPMI 8226, and U87-MG. FIG. 17A shows the results of an in vitro killing experiment of dual VHH CAR-T against K562 and K562-BCMA. The results show that all the four types of CAR-T cells had specific killing effects on K562-BCMA, and when the effector-to-target ratio is 3:1, the killing ratio of dual VHH CAR-T was higher than that of single VHH CAR-T. FIG. 17B shows the results of an in vitro killing experiment of dual VHH CAR-T against RPMI 8226 and U87-MG. The results show that dual VHH CAR-T also had specific killing effects on the BCMA-positive cell line RPMI 8226, but had no obvious killing effect on the BCMA-negative cell line U87-MG.

Moreover, the concentration of INF-γ in the culture supernatant obtained after the dual VHH CAR-T cells were respectively co-incubated with K562-BCMA and RPMI 8226 was much higher than that after incubation with U87-MG (see FIG. 18). The above results show that the dual VHH CAR-T cells had a high killing effect on the BCMA-positive cell lines, and had no killing effect on the BCMA-negative cell lines, indicating that the killing effects of the two types of dual VHH CAR-T cells against BCMA-positive cell lines are specific.

### Example 5 Identification of antibody characteristics

### 1. Recombinant antibody expression

To further determine the characteristics of the nanobodies Nb003-01 and Nb003-02 and the dual nanobodies dNb003-A and dNb003-B screened in Example 1, the nanobodies were expressed in vitro and purified in this example. First, the nanobodies Nb003-01, Nb003-02, dNb003-A, and dNb003-B were inserted into the BamH I and NHE I cleavage sites of the pET-28a-Sumo-Nb-Fc plasmid (retained in this laboratory) by molecular cloning. The N-terminus of the nanobody sequence comprised a SUMO tag (SUMO tag protein is a small molecule ubiquitin-like modified protein. Studies have found that SUMO can be used as a fusion tag and molecular chaperone for recombinant protein expression, which can not only further increase the expression of the fusion protein, but also has the functions of resisting protease hydrolysis, promoting the correct folding of the target protein, and improving the solubility of the recombinant protein). The C-terminus of the nanobody sequence carried a human IgG1 Fc tag for protein purification. The plasmid sequenced to be correct was extracted and then transformed into the *Escherichia coli* strain BL21, and the protein was expressed under IPTG induction. After the expression was completed, the bacteria were collected and ultrasonically lysed to obtain crude protein. The crude protein was purified by Protein A affinity chromatography to obtain nanobodies with high purity. The nanobodies obtained by purification were named Nb003-01-Fc, Nb003-02-Fc, dNb003-A-Fc, and dNb003-B-Fc.

### 2. Affinity assay

### (1) Detection of antibody affinity by ELISA

In this example, the ELISA detection method was used to measure the affinity of nanobodies Nb003-01-Fc, Nb003-02-Fc, dNb003-A-Fc, and dNb003-B-Fc for human BCMA. The BCMA-His protein prepared in Example 1 was coated overnight, and the affinity between each antibody and the corresponding antigen was detected using the nanobodies of different dilutions after expression and purification as the primary antibody and the anti-human IgG1 Fc-HRP antibody as the secondary antibody.

### (2) Detection of antibody affinity by SPR

The expressed and purified nanobodies Nb003-01-Fc, Nb003-02-Fc, and dNb003-B-Fc were used for SPR measurement. The affinity of nanobodies Nb003-01-Fc, Nb003-02-Fc, and dNb003-B-Fc for human BCMA was measured by SPR using a Biacore 8K analysis system.

A method for detecting the affinity between each nanobody and BCMA-His was as follows. First, BCMA-His was fixed on an NTA chip using a capture method. Then, Nb003-01-Fc, Nb003-02-Fc, and dNb003-B-Fc diluted in multiple ratios were injected as mobile phases at a flow rate of 30 µL/min, and then dissociated. Finally, the results were analyzed using the Biacore T200 Evaluation Software Kinetic 1:1 Binding mode.

### 3. Specificity detection method

The specificity of Nb003-01, Nb003-02, dNb003-A, and dNb003-B for BCMA was detected by flow cytometry. The specificity of Nb003-01-Fc, Nb003-02-Fc, dNb003-A-Fc, and dNb003-B-Fc for BCMA-positive cell lines was detected by flow cytometry. The binding of each nanobody to human BCMA-positive cell lines K562-BCMA, RPMI 8226, and BCMA-negative cell line K562 was detected by flow cytometry using the biotinylated purified nanobodies Nb003-01-Fc, Nb003-02-Fc, dNb003-A-Fc, and dNb003-B-Fc as primary antibodies and SA-PE antibody as the secondary antibody. As such, the specificity of each nanobody for human BCMA antigen was determined.

### 4. Experimental results

The results of antibody affinity activity detected by ELISA method are shown in FIG. 19. The results show that the EC50 value of Nb003-01-Fc against human BCMA is 2.048 nM, the EC50 value of Nb003-02-Fc against human BCMA was 0.6518 nM, the EC50 value of dNb003-A-Fc against human BCMA was 0.02227 nM, and the EC50 value of dNb003-B-Fc against human BCMA was 0.3458 nM.

The results of antibody affinity activity detected by SPR method are shown in Table 4 and FIG. 20. The results show that the affinity constant between Nb003-01 and human BCMA was 6.20×10⁻⁸; the affinity constant between Nb003-02 and human BCMA is 1.79×10⁻⁹; and the affinity constant between dNb003-B and human BCMA is 1.94×10⁻⁹.

The above results show that the two anti-BCMA nanobodies Nb003-01 and Nb003-02 obtained by screening and identification in the present application had good affinity with human BCMA; and the dual nanobodies dNb003-B had good affinity with human BCMA, which tended to be higher than that of a single nanobody.

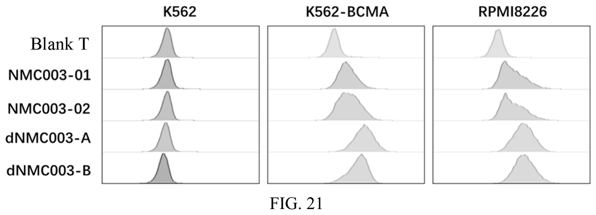

The flow cytometry results show that Nb003-01-Fc, Nb003-02-Fc, dNb003-A-Fc, and dNb003-B-Fc did not specifically bind to K562, but all specifically bound to BCMA-positive cell lines K562-BCMA and RPMI 8226 (see FIG. 21), proving that Nb003-01-Fc, Nb003-02-Fc, dNb003-A-Fc, and dNb003-B-Fc are specific antibodies for BCMA.

**Table 4 Statistical results of detection of antibody affinity by SPR**

| Ligand | Analyte | Binding rate constant ka (1/Ms) | Dissociation rate constant kd (1/s) | Affinity KD (M) |
|---|---|---|---|---|
| Human BCMA | Nb003-01-Fc | 1.38×10⁴ | 8.57×10⁻⁴ | 6.20×10⁻⁸ |
| Human BCMA | Nb003-02-Fc | 1.19×10⁶ | 2.12×10⁻³ | 1.79×10⁻⁹ |
| Human BCMA | dNb003-B-Fc | 1.27×10⁶ | 2.45×10⁻³ | 1.94×10⁻⁹ |

The description of the above embodiments is merely for understanding the method of the present application and the core idea thereof, and those skilled in the art can also make several improvements and modifications to the present application, which will also fall within the scope of protection of the claims of the present application.

## Claims

1. A nanobody targeting BCMA, wherein the nanobody comprises VHH01 or VHH02,
wherein
amino acid sequences of CDR1, CDR2, and CDR3 of the VHH01 are as shown in SEQ ID NO: 3, SEQ ID NO: 5, and SEQ ID NO: 7, respectively;
amino acid sequences of CDR1, CDR2, and CDR3 of the VHH02 are as shown in SEQ ID NO: 11, SEQ ID NO: 13, and SEQ ID NO: 15, respectively.

2. The nanobody according to claim 1, wherein nucleotide sequences of the CDR1, CDR2, and CDR3 of the VHH01 are as shown in SEQ ID NO: 4, SEQ ID NO: 6, and SEQ ID NO: 8, respectively.

3. The nanobody according to claim 2, wherein the amino acid sequence of the VHH01 is as shown in SEQ ID NO: 1.

4. The nanobody according to claim 3, wherein the nucleotide sequence of the VHH01 is as shown in SEQ ID NO: 2.

5. The nanobody according to claim 1, wherein nucleotide sequences of the CDR1, CDR2, and CDR3 of the VHH02 are as shown in SEQ ID NO: 12, SEQ ID NO: 14, and SEQ ID NO: 16, respectively.

6. The nanobody according to claim 5, wherein an amino acid sequence of the VHH02 is as shown in SEQ ID NO: 9.

7. The nanobody according to claim 6, wherein a nucleotide sequence of the VHH02 is as shown in SEQ ID NO: 10.

8. A single-nanobody-based chimeric antigen receptor targeting BCMA, wherein the chimeric antigen receptor comprises the nanobody according to any one of claims 1 to 7.

9. The chimeric antigen receptor according to claim 8, wherein the chimeric antigen receptor further comprises a transmembrane domain.

10. The chimeric antigen receptor according to claim 9, wherein the chimeric antigen receptor further comprises an intracellular signaling domain.

11. The chimeric antigen receptor according to claim 10, wherein the chimeric antigen receptor further comprises a hinge region.

12. The chimeric antigen receptor according to claim 11, wherein the chimeric antigen receptor further comprises a signal peptide.

13. The chimeric antigen receptor according to claim 12, wherein the chimeric antigen receptor further comprises a costimulatory domain.

14. The chimeric antigen receptor according to claim 13, wherein the chimeric antigen receptor further comprises a promoter.

15. The chimeric antigen receptor according to claim 14, wherein the chimeric antigen receptor further comprises a self-cleaving peptide.

16. The chimeric antigen receptor according to claim 15, wherein the chimeric antigen receptor further comprises a detection tag/auxiliary function element.

17. The chimeric antigen receptor according to claim 16, wherein the chimeric antigen receptor further comprises a tEGFR signal peptide.

18. The chimeric antigen receptor according to claim 9, wherein the transmembrane domain comprises a transmembrane domain of the following molecules: CD8α, CD28, 4-1BB, CD34, CD3ε, PD-1, IgG1, IgG4, OX40, IL-2 receptor, IL-7 receptor, or IL-11 receptor.

19. The chimeric antigen receptor according to claim 10, wherein the intracellular signaling domain comprises an intracellular signaling domain of the following molecules: CD3ζ, CD3γ, CD3δ, CD3ε, FcRγ, FcRβ, TCRζ, CD4, CD5, CD8, CD21, CD22, CD79a, CD79b, CD278, FcεRI, DAP10, DAP12, or CD66d.

20. The chimeric antigen receptor according to claim 11, wherein the hinge region comprises a hinge region of the following molecules: CD8α, CD28, CD34, 4-1BB, OX40, CD3ε, IgG1, IgG4, PD-1, IL-2 receptor, IL-7 receptor, or IL-11 receptor.

21. The chimeric antigen receptor according to claim 12, wherein the signal peptide comprises a signal peptide of the following molecules: T cell receptor α- and β- chains, CD3ζ, CD3ε, CD16, CD22, CD33, CD4, CD5, CD8, CD9, CD28, CD37, CD45, CD64, CD80, CD86, CD134, CD137, CD154, GITR, or GM-CSF.

22. The chimeric antigen receptor according to claim 13, wherein the costimulatory domain comprises a costimulatory domain of the following molecules: 4-1BB(CD137), CD19, CD4, CD27, CD28, ICOS(CD278), CD8α, CD8β, BAFFR, HVEM, LIGHT, KIRDS2, SLAMF7, NKp30, NKp46, CD40, CDS, ICAM-1, B7-H3, OX40, DR3, GITR, CD30, TIM1, CD2, CD7, or CD226.

23. The chimeric antigen receptor according to claim 14, wherein the promoter comprises: EF1α promoter, CMV promoter, EFS promoter, CAG promoter, CBh promoter, SFFV promoter, MSCV promoter, SV40 promoter, mPGK promoter, hPGK promoter, or UBC promoter.

24. The chimeric antigen receptor according to claim 15, wherein the self-cleaving peptide comprises: T2A, P2A, E2A, or F2A.

25. The chimeric antigen receptor according to claim 16, wherein the detection tag/auxiliary function element comprises: tEGFR, tCD34, tCD19, tCD20, tCD22, immune checkpoint inhibitor (CTLA-4, PD-1/PD-L1, LAG-3, TIM-3, TIGIT, CD226, CD155, CD47, B7-H3, or B7-H4) nanobodies, cytokines or receptors thereof (IL2, IL2 receptor, IL7, IL7 receptor, IL15, or IL15 receptor).

26. The chimeric antigen receptor according to claim 18, wherein the transmembrane domain is a CD8a transmembrane domain.

27. The chimeric antigen receptor according to claim 19, wherein the intracellular signaling domain is a CD3ζ intracellular signaling domain.

28. The chimeric antigen receptor according to claim 20, wherein the hinge region is a CD8a hinge region.

29. The chimeric antigen receptor according to claim 22, wherein the costimulatory domain is a 4-1BB costimulatory domain.

30. The chimeric antigen receptor according to claim 23, wherein the promoter is EF1α.

31. The chimeric antigen receptor according to claim 24, wherein the self-cleaving peptide is T2A.

32. The chimeric antigen receptor according to claim 25, wherein the detection tag/auxiliary function element is tEGFR.

33. The chimeric antigen receptor according to claim 32, wherein the chimeric antigen receptor is obtained by connecting EF1α, a signal peptide, the nanobody according to any one of claims 1 to 7, a CD8α hinge region, a CD8a transmembrane domain, a 4-1BB costimulatory domain, a CD3ζ intracellular signaling domain, T2A, a tEGFR signal peptide, and tEGFR sequentially.

34. The chimeric antigen receptor according to claim 33, wherein the chimeric antigen receptor is obtained by connecting EF1α, a signal peptide, VHH01, a CD8a hinge region, a CD8a transmembrane domain, a 4-1BB costimulatory domain, a CD3ζ intracellular signaling domain, T2A, a tEGFR signal peptide, and tEGFR sequentially.

35. The chimeric antigen receptor according to claim 33, wherein the chimeric antigen receptor is obtained by connecting EF1α, a signal peptide, VHH02, a CD8a hinge region, a CD8α transmembrane domain, a 4-1BB costimulatory domain, a CD3ζ intracellular signaling domain, T2A, a tEGFR signal peptide, and tEGFR sequentially.

36. A dual-nanobody-based chimeric antigen receptor targeting BCMA, wherein the chimeric antigen receptor comprises any one of the nanobody according to any one of claims 1 to 7 and any another nanobody targeting BCMA.

37. The chimeric antigen receptor according to claim 36, wherein the any another nanobody targeting BCMA is VHH02.

38. The chimeric antigen receptor according to claim 37, wherein amino acid sequences of the CDR1, CDR2, and CDR3 of the VHH02 are as shown in SEQ ID NO: 11, SEQ ID NO: 13, and SEQ ID NO: 15, respectively.

39. The chimeric antigen receptor according to claim 38, wherein nucleotide sequences of the CDR1, CDR2, and CDR3 of the VHH02 are as shown in SEQ ID NO: 12, SEQ ID NO: 14, and SEQ ID NO: 16, respectively.

40. The chimeric antigen receptor according to claim 39, wherein an amino acid sequence of the VHH02 is as shown in SEQ ID NO: 9.

41. The chimeric antigen receptor according to claim 40, wherein a nucleotide sequence of the VHH02 is as shown in SEQ ID NO: 10.

42. The chimeric antigen receptor according to claim 41, wherein the chimeric antigen receptor further comprises a transmembrane domain.

43. The chimeric antigen receptor according to claim 42, wherein the chimeric antigen receptor further comprises an intracellular signaling domain.

44. The chimeric antigen receptor according to claim 43, wherein the chimeric antigen receptor further comprises a hinge region.

45. The chimeric antigen receptor according to claim 44, wherein the chimeric antigen receptor further comprises a signal peptide.

46. The chimeric antigen receptor according to claim 45, wherein the chimeric antigen receptor further comprises a costimulatory domain.

47. The chimeric antigen receptor according to claim 46, wherein the chimeric antigen receptor further comprises a promoter.

48. The chimeric antigen receptor according to claim 47, wherein the chimeric antigen receptor further comprises a self-cleaving peptide.

49. The chimeric antigen receptor according to claim 48, wherein the chimeric antigen receptor further comprises a detection tag/auxiliary function element.

50. The chimeric antigen receptor according to claim 49, wherein the chimeric antigen receptor further comprises a tEGFR signal peptide.

51. The chimeric antigen receptor according to claim 42, wherein the transmembrane domain comprises a transmembrane domain of the following molecules: CD8α, CD28, 4-1BB, CD34, CD3ε, PD-1, IgG1, IgG4, OX40, IL-2 receptor, IL-7 receptor, or IL-11 receptor.

52. The chimeric antigen receptor according to claim 43, wherein the intracellular signaling domain comprises an intracellular signaling domain of the following molecules: CD3ζ, CD3γ, CD3δ, CD3ε, FcRγ, FcRβ, TCRζ, CD4, CD5, CD8, CD21, CD22, CD79a, CD79b, CD278, FcεRI, DAP10, DAP12, or CD66d.

53. The chimeric antigen receptor according to claim 44, wherein the hinge region comprises a hinge region of the following molecules: CD8α, CD28, CD34, 4-1BB, OX40, CD3ε, IgG1, IgG4, PD-1, IL-2 receptor, IL-7 receptor, or IL-11 receptor.

54. The chimeric antigen receptor according to claim 45, wherein the signal peptide comprises a signal peptide of the following molecules: T cell receptor α- and β- chains, CD3ζ, CD3ε, CD16, CD22, CD33, CD4, CD5, CD8, CD9, CD28, CD37, CD45, CD64, CD80, CD86, CD134, CD137, CD154, GITR, or GM-CSF.

55. The chimeric antigen receptor according to claim 46, wherein the costimulatory domain comprises a costimulatory domain of the following molecules: 4-1BB(CD137), CD19, CD4, CD27, CD28, ICOS(CD278), CD8α, CD8β, BAFFR, HVEM, LIGHT, KIRDS2, SLAMF7, NKp30, NKp46, CD40, CDS, ICAM-1, B7-H3, OX40, DR3, GITR, CD30, TIM1, CD2, CD7, or CD226.

56. The chimeric antigen receptor according to claim 47, wherein the promoter comprises: EF1α promoter, CMV promoter, EFS promoter, CAG promoter, CBh promoter, SFFV promoter, MSCV promoter, SV40 promoter, mPGK promoter, hPGK promoter, or UBC promoter.

57. The chimeric antigen receptor according to claim 48, wherein the self-cleaving peptide comprises: T2A, P2A, E2A, or F2A.

58. The chimeric antigen receptor according to claim 49, wherein the detection tag/auxiliary function element comprises: tEGFR, tCD34, tCD19, tCD20, tCD22, immune checkpoint inhibitor (CTLA-4, PD-1/PD-L1, LAG-3, TIM-3, TIGIT, CD226, CD155, CD47, B7-H3, or B7-H4) nanobodies, cytokines or receptors thereof (IL2, IL2 receptor, IL7, IL7 receptor, IL15, or IL15 receptor).

59. The chimeric antigen receptor according to claim 51, wherein the transmembrane domain is a CD8a transmembrane domain.

60. The chimeric antigen receptor according to claim 52, wherein the intracellular signaling domain is a CD3ζ intracellular signaling domain.

61. The chimeric antigen receptor according to claim 53, wherein the hinge region is a CD8a hinge region.

62. The chimeric antigen receptor according to claim 55, wherein the costimulatory domain is a 4-1BB costimulatory domain.

63. The chimeric antigen receptor according to claim 56, wherein the promoter is EF1α.

64. The chimeric antigen receptor according to claim 57, wherein the self-cleaving peptide is T2A.

65. The chimeric antigen receptor according to claim 58, wherein the detection tag/auxiliary function element is tEGFR.

66. The chimeric antigen receptor according to claim 65, wherein the chimeric antigen receptor is obtained by connecting EF1α, a signal peptide, any one of the nanobody according to any one of claims 1 to 7, a linker, any another nanobody targeting BCMA, a CD8a hinge region, a CD8α transmembrane domain, a 4-1BB costimulatory domain, a CD3ζ intracellular signaling domain, T2A, a tEGFR signal peptide, and tEGFR sequentially.

67. The chimeric antigen receptor according to claim 65, wherein the chimeric antigen receptor is obtained by connecting EF1α, a signal peptide, any another nanobody targeting BCMA, a linker, any one of the nanobody according to any one of claims 1 to 7, a CD8a hinge region, a CD8a transmembrane domain, a 4-1BB costimulatory domain, a CD3ζ intracellular signaling domain, T2A, a tEGFR signal peptide, and tEGFR sequentially.

68. The chimeric antigen receptor according to claim 66, wherein the chimeric antigen receptor is obtained by connecting EF1α, a signal peptide, VHH01, a linker, VHH02, a CD8a hinge region, a CD8a transmembrane domain, a 4-1BB costimulatory domain, a CD3ζ intracellular signaling domain, T2A, a tEGFR signal peptide, and tEGFR sequentially.

69. The chimeric antigen receptor according to claim 67, wherein the chimeric antigen receptor is obtained by connecting EF1α, a signal peptide, VHH02, a linker, VHH02, a CD8a hinge region, a CD8a transmembrane domain, a 4-1BB costimulatory domain, a CD3ζ intracellular signaling domain, T2A, a tEGFR signal peptide, and tEGFR sequentially.

70. The chimeric antigen receptor according to claim 69, wherein the linker is (G4S)₅.

71. An isolated nucleic acid molecule, comprising a nucleotide sequence encoding the nanobody according to any one of claims 1 to 7, the chimeric antigen receptor according to any one of claims 8 to 35, or the chimeric antigen receptor according to any one of claims 36 to 70.

72. The nucleic acid molecule according to claim 71, wherein the nucleotide sequence of VHH01 in the nanobody according to any one of claims 1 to 7 is as shown in SEQ ID NO: 2.

73. The nucleic acid molecule according to claim 71, wherein the nucleotide sequence of VHH02 in the nanobody according to any one of claims 1 to 7 is as shown in SEQ ID NO: 10.

74. The nucleic acid molecule according to claim 71, wherein the nucleotide sequence of EF1a in the chimeric antigen receptor according to any one of claims 8 to 35 or the chimeric antigen receptor according to any one of claims 36 to 70 is as shown in SEQ ID NO: 17.

75. The nucleic acid molecule according to claim 71, wherein the nucleotide sequence of a signal peptide in the chimeric antigen receptor according to any one of claims 8 to 35 or the chimeric antigen receptor according to any one of claims 36 to 70 is as shown in SEQ ID NO: 26.

76. The nucleic acid molecule according to claim 71, wherein the nucleotide sequence of a CD8a hinge region in the chimeric antigen receptor according to any one of claims 8 to 35 or the chimeric antigen receptor according to any one of claims 36 to 70 is as shown in SEQ ID NO: 27.

77. The nucleic acid molecule according to claim 71, wherein the nucleotide sequence of a CD8a transmembrane domain in the chimeric antigen receptor according to any one of claims 8 to 35 or the chimeric antigen receptor according to any one of claims 36 to 70 is as shown in SEQ ID NO: 28.

78. The nucleic acid molecule according to claim 71, wherein the nucleotide sequence of a 4-1BB costimulatory signal domain in the chimeric antigen receptor according to any one of claims 8 to 35 or the chimeric antigen receptor according to any one of claims 36 to 70 is as shown in SEQ ID NO: 29.

79. The nucleic acid molecule according to claim 71, wherein the nucleotide sequence of a CD3ζ intracellular signaling domain in the chimeric antigen receptor according to any one of claims 8 to 35 or the chimeric antigen receptor according to any one of claims 36 to 70 is as shown in SEQ ID NO: 30.

80. The nucleic acid molecule according to claim 71, wherein the nucleotide sequence of T2A in the chimeric antigen receptor according to any one of claims 8 to 35 or the chimeric antigen receptor according to any one of claims 36 to 70 is as shown in SEQ ID NO: 31.

81. The nucleic acid molecule according to claim 71, wherein the nucleotide sequence of a tEGFR signal peptide in the chimeric antigen receptor according to any one of claims 8 to 35 or the chimeric antigen receptor according to any one of claims 36 to 70 is as shown in SEQ ID NO: 32.

82. The nucleic acid molecule according to claim 71, wherein the nucleotide sequence of tEGFR in the chimeric antigen receptor according to any one of claims 8 to 35 or the chimeric antigen receptor according to any one of claims 36 to 70 is as shown in SEQ ID NO: 33.

83. An expression vector comprising the nucleic acid molecule according to claim 71.

84. The expression vector according to claim 83, wherein the vector comprises a DNA vector, an RNA vector, a plasmid, or a vector derived from virus.

85. The expression vector according to claim 84, wherein the vector derived from virus comprises a lentiviral vector, a retroviral vector, an adenoviral vector, an adeno-associated virus vector, a poxvirus vector, or a herpes virus vector.

86. An engineered host cell, comprising the expression vector according to claim 83.

87. The engineered host cell according to claim 86, wherein the host cell is a eukaryotic cell or a prokaryotic cell.

88. The engineered host cell according to claim 87, wherein the host cell is an immune cell.

89. The engineered host cell according to claim 88, wherein the immune cell comprises T cells, B cells, NK cells, iNKT cells, CTL cells, dendritic cells, myeloid cells, monocytes, macrophages, or any combination thereof.

90. The engineered host cell according to claim 89, wherein the immune cell is a T cell.

91. An immunoconjugate, comprising the nanobody according to any one of claims 1 to 7 and a conjugate moiety conjugated to the nanobody.

92. The immunoconjugate according to claim 91, wherein the conjugate moiety comprises a detectable marker, a radionuclide, a cytokine, a therapeutic agent, a cytotoxin, an enzyme, a gold nanoparticle/nanorod, a nanomagnetic particle, a viral coat protein, a virus-like particle (VLP), or a combination thereof.

93. The immunoconjugate according to claim 92, wherein the detectable marker comprises a fluorescent or luminescent label, a radioactive label, or a magnetic resonance imaging (MRI) or CT (X-ray computed tomography) contrast agent.

94. The immunoconjugate according to claim 92, wherein the radionuclide comprises ¹³¹I, ³²P, ⁸⁹Sr, ⁹⁰Y, ²²³Ra, ¹²⁵I or ¹⁰³Pd.

95. The immunoconjugate according to claim 92, wherein the cytokine comprises IL-2, IL-3, IL-4, IL-5, IL-6, IL-9, IL-10, IL-12, IL-13, IL-14, IFN-γ, TNF-β, TNF-α, G-CSF or M-CSF.

96. The immunoconjugate according to claim 92, wherein the therapeutic agent comprises an alkylating agent, an antimetabolite, an anti-tumor antibiotic, a mitotic inhibitor, a chromatin function inhibitor, an anti-angiogenic agent, an anti-estrogen, an anti-androgen or an immunomodulator.

97. The immunoconjugate according to claim 96, wherein the alkylating agent comprises bis-chloroethyl methylamine, chlorambucil, phenylalanine mustard, pipobroman, prednimustine, estramustine phosphate, cyclophosphamide, hexamethylmelamine, ifosfamide, fortepidine, thiotepa, carmustine, streptozotocin, improsulfan, dacarbazine, cisplatin, oxaliplatin or carboplatin.

98. The immunoconjugate according to claim 96, wherein the antimetabolite comprises methotrexate, 5-fluorouracil, floxuridine, 5-fluorodeoxyuracil, capecitabine, cytarabine, fludarabine, 6-mercaptopurine, 2-chlorodeoxyadenosine, 5-azacytidine, 2,2-difluorodeoxycytidine, cladribine, deoxycoformycin or pentostatin.

99. The immunoconjugate according to claim 96, wherein the anti-tumor antibiotic comprises daunorubicin, doxorubicin, idarubicin, plicamycin, mitomycin C, valrubicin, mitoxantrone hydrochloride, bleomycin, dactinomycin, mithramycin A or procarbazine.

100. The immunoconjugate according to claim 96, wherein the mitotic inhibitor comprises docetaxel, vinblastine, paclitaxel, vincristine, vindesine or vinorelbine.

101. The immunoconjugate according to claim 96, wherein the chromatin function inhibitor comprises irinotecan, etoposide, topotecan, etoposide phosphate or teniposide.

102. The immunoconjugate according to claim 96, wherein the anti-angiogenic agent comprises prinomastat, tanomastat, ilomastat, razoxane, marimastat, batimastat, CGS-27023A, halofuginone, COL-3, neovastat, BMS-275291 or thalidomide.

103. The immunoconjugate according to claim 96, wherein the anti-estrogen comprises toremifene, raloxifene, tamoxifen, anastrozole, letrozole, droloxifene, odoxifene or exemestane.

104. The immunoconjugate according to claim 96, wherein the anti-androgen comprises nilutamide, bicalutamide, spironolactone, flutamide, finasteride, cyproterone acetate or cimetidine.

105. The immunoconjugate according to claim 96, wherein the immunomodulator comprises interleukin, tumor necrosis factor, interferon, mushroom polysaccharide, sizofiran, roquinimex, pidotimod, methoxypolyethylene glycol succinamide adenosine deaminase or a thymosin preparation.

106. The immunoconjugate according to claim 92, wherein the cytotoxin comprises MMAE, DM1, Ozogamicin, Dxd, SN-38, MMAF, PBD, DM2, Amanitin, DM4, PNU-159682, IR700, PE-38, PE24, PE-T20, PE-T20-KDEL, PE4E or PE40.

107. A reagent for detecting a BCMA protein, comprising the nanobody according to any one of claims 1 to 7 or the immunoconjugate according to claim 91.

108. The reagent according to claim 107, wherein the reagent further comprises a diagnostic agent coupled to the nanobody or the immunoconjugate.

109. The reagent according to claim 108, wherein the diagnostic agents comprise a radionuclide, a chemiluminescent agent, a bioluminescent agent, a paramagnetic ion, an enzyme or a photosensitive diagnostic agent.

110. The reagent according to claim 109, wherein the radionuclide comprises ¹⁸F, ⁵²Fe, ⁶²Cu, ⁶⁴Cu, ⁶⁷Cu, ⁸⁶Y, ⁹⁰Y, ⁸⁹Zr, ¹²⁰I, ¹²³I, ¹²⁴I, ¹²⁵I, ¹³¹I, ¹³N, ¹⁵O, ¹⁸⁶Re, ¹⁸⁸Re, ⁵¹Mn, ⁵⁵Co or ⁷²As.

111. The reagent according to claim 109, wherein the chemiluminescent agent comprises luminol, isoluminol, an aromatic acridinium ester, an imidazole, an acridinium salt or an oxalate ester.

112. The reagent according to claim 109, wherein the bioluminescent agent comprises fluorescein, luciferase or aequorin.

113. The reagent according to claim 109, wherein the paramagnetic ion comprises chromium (III), manganese (II), iron (III), iron (II), cobalt (II), nickel (II), copper (II), neodymium (III), samarium (III), ytterbium (III), gadolinium (III), vanadium (II), terbium (III), dysprosium (III), holmium (III) or erbium (III).

114. The reagent according to claim 109, wherein the enzyme comprises horseradish peroxidase, alkaline phosphatase, glucose oxidase, β-D-galactosidase, urease, catalase, or glucoamylase.

115. The reagent according to claim 109, wherein the photosensitive diagnostic agent comprises dihydroxysilicon phthalocyanine, methylene blue, protoporphyrin, hematoporphyrin or photofrin.

116. A pharmaceutical composition, comprising the nanobody according to any one of claims 1 to 7, the engineered host cell according to claim 86, and/or the immunoconjugate according to claim 91.

117. The pharmaceutical composition according to claim 116, wherein the pharmaceutical composition further comprises one or more pharmaceutically or physiologically acceptable carriers and/or excipients.

118. The pharmaceutical composition according to claim 117, wherein the pharmaceutical composition is used for treating and/or preventing a BCMA-positive related disease.

119. The pharmaceutical composition according to claim 118, wherein the BCMA-positive related disease comprises multiple myeloma, acute myeloid leukemia, chronic myeloid leukemia, chronic lymphocytic leukemia, acute lymphocytic leukemia, diffuse large B-cell lymphoma, Hodgkin's lymphoma, non-Hodgkin's lymphoma, gastric cancer, liver cancer, kidney cancer, lung cancer, small intestine cancer, bone cancer, prostate cancer, colorectal cancer, breast cancer, bowel cancer, cervical cancer, ovarian cancer, lymphoma, nasopharyngeal carcinoma, adrenal tumor, bladder tumor, non-small cell lung cancer, brain glioma, endometrial cancer, fibrosarcoma, myxosarcoma, liposarcoma mesothelioma, lymphoid malignancies or pancreatic cancer.

120. A kit comprising the nanobody according to any one of claims 1 to 7, the immunoconjugate according to claim 91, and/or the reagent according to claim 107.

121. The kit according to claim 120, wherein the kit is used for detecting a BCMA protein.

122. A method for producing the nanobody according to any one of claims 1 to 7, comprising the following steps:
(1) culturing the engineered host cell according to claim 86 under conditions suitable for production of the nanobody, to obtain a culture containing the nanobody;
(2) isolating or recovering the nanobody from the culture obtained in the step (1); and
(3) purifying the nanobody obtained in the step (2) to obtain the nanobody according to any one of claims 1 to 7.

123. A method for preparing the engineered host cell according to claim 86, wherein the method comprises the following step: introducing the nucleic acid molecule according to claim 71 or the expression vector according to claim 83 into a host cell.

124. The method according to claim 123, wherein the method of introduction comprises lipid transfection, microinjection, electroporation, DNA vector, RNA vector, retroviral vector, lentiviral vector, poxvirus vector, herpes simplex virus vector, adenovirus vector, or adeno-associated virus vector.

125. The method according to claim 123, wherein the host cell is a eukaryotic cell or a prokaryotic cell.

126. The method according to claim 125, wherein the host cell is an immune cell.

127. The method according to claim 126, wherein the immune cell comprises T cells, B cells, NK cells, iNKT cells, CTL cells, dendritic cells, myeloid cells, monocytes, macrophages, or any combination thereof.

128. The method according to claim 127, wherein the immune cell is a T cell.

129. A method for detecting a BCMA protein, the method comprising: contacting a sample to be tested with the nanobody according to any one of claims 1 to 7, the immunoconjugate according to claim 91, and/or the reagent according to claim 107; and detecting presence of an antibody-antigen complex.

130. A method for diagnosing whether a subject has a BCMA-positive related disease, wherein the method comprises the following steps: contacting a sample to be tested from the subject with the nanobody according to any one of claims 1 to 7, the immunoconjugate according to claim 91, and/or the reagent according to claim 107; and detecting formation of a complex between the nanobody, the immunoconjugate, and/or the reagent and BCMA or the amount of the complex,
wherein the formation of the complex indicates that BCMA or a BCMA-expressing cell is present, and the subject has a BCMA-positive related disease.

131. A method for treating a BCMA-positive related disease, wherein the method comprises administering an effective amount of the nanobody according to any one of claims 1 to 7, the engineered host cell according to claim 86, the immunoconjugate according to claim 91, and/or the pharmaceutical composition according to claim 116 to a subject in need thereof.

132. The method according to claim 131, wherein routes of the administration comprise parenteral, intrapulmonary, or intranasal administration.

133. Use of the nanobody according to any one of claims 1 to 7 for detecting a BCMA protein and/or diagnosing a BCMA-positive related disease.

134. The use according to claim 133, wherein the BCMA-positive related disease comprises multiple myeloma, acute myeloid leukemia, chronic myeloid leukemia, chronic lymphocytic leukemia, acute lymphocytic leukemia, diffuse large B-cell lymphoma, Hodgkin's lymphoma, non-Hodgkin's lymphoma, gastric cancer, liver cancer, kidney cancer, lung cancer, small intestine cancer, bone cancer, prostate cancer, colorectal cancer, breast cancer, bowel cancer, cervical cancer, ovarian cancer, lymphoma, nasopharyngeal carcinoma, adrenal tumor, bladder tumor, non-small cell lung cancer, brain glioma, endometrial cancer, fibrosarcoma, myxosarcoma, liposarcoma mesothelioma, lymphoid malignancies, or pancreatic cancer.

135. Use of the nanobody according to any one of claims 1 to 7 in the manufacture of a reagent or kit for detecting a BCMA protein and/or diagnosing a BCMA-positive related disease.

136. The use according to claim 135, wherein the BCMA-positive related disease comprises multiple myeloma, acute myeloid leukemia, chronic myeloid leukemia, chronic lymphocytic leukemia, acute lymphocytic leukemia, diffuse large B-cell lymphoma, Hodgkin's lymphoma, non-Hodgkin's lymphoma, gastric cancer, liver cancer, kidney cancer, lung cancer, small intestine cancer, bone cancer, prostate cancer, colorectal cancer, breast cancer, bowel cancer, cervical cancer, ovarian cancer, lymphoma, nasopharyngeal carcinoma, adrenal tumor, bladder tumor, non-small cell lung cancer, brain glioma, endometrial cancer, fibrosarcoma, myxosarcoma, liposarcoma mesothelioma, lymphoid malignancies, or pancreatic cancer.

137. Use of the reagent according to claim 107 or the kit according to claim 120 for detecting a BCMA protein and/or diagnosing a BCMA-positive related disease.

138. The use according to claim 137, wherein the BCMA-positive related disease comprises multiple myeloma, acute myeloid leukemia, chronic myeloid leukemia, chronic lymphocytic leukemia, acute lymphocytic leukemia, diffuse large B-cell lymphoma, Hodgkin's lymphoma, non-Hodgkin's lymphoma, gastric cancer, liver cancer, kidney cancer, lung cancer, small intestine cancer, bone cancer, prostate cancer, colorectal cancer, breast cancer, bowel cancer, cervical cancer, ovarian cancer, lymphoma, nasopharyngeal carcinoma, adrenal tumor, bladder tumor, non-small cell lung cancer, brain glioma, endometrial cancer, fibrosarcoma, myxosarcoma, liposarcoma mesothelioma, lymphoid malignancies, or pancreatic cancer.

139. Use of the nanobody according to any one of claims 1 to 7, the chimeric antigen receptor according to claim 8, the chimeric antigen receptor according to claim 36, the nucleic acid molecule according to claim 71, or the expression vector according to claim 83 in the manufacture of an engineered host cell for treating and/or preventing a BCMA-positive related disease.

140. The use according to claim 139, wherein the BCMA-positive related disease comprises multiple myeloma, acute myeloid leukemia, chronic myeloid leukemia, chronic lymphocytic leukemia, acute lymphocytic leukemia, diffuse large B-cell lymphoma, Hodgkin's lymphoma, non-Hodgkin's lymphoma, gastric cancer, liver cancer, kidney cancer, lung cancer, small intestine cancer, bone cancer, prostate cancer, colorectal cancer, breast cancer, bowel cancer, cervical cancer, ovarian cancer, lymphoma, nasopharyngeal carcinoma, adrenal tumor, bladder tumor, non-small cell lung cancer, brain glioma, endometrial cancer, fibrosarcoma, myxosarcoma, liposarcoma mesothelioma, lymphoid malignancies, or pancreatic cancer.

141. Use of the nanobody according to any one of claims 1 to 7, the chimeric antigen receptor according to claim 8, the chimeric antigen receptor according to claim 36, the nucleic acid molecule according to claim 71, the expression vector according to claim 83, or the engineered host cell according to claim 86 in the manufacture of an immunoconjugate for treating and/or preventing a BCMA-positive related disease.

142. The use according to claim 141, wherein the BCMA-positive related disease comprises multiple myeloma, acute myeloid leukemia, chronic myeloid leukemia, chronic lymphocytic leukemia, acute lymphocytic leukemia, diffuse large B-cell lymphoma, Hodgkin's lymphoma, non-Hodgkin's lymphoma, gastric cancer, liver cancer, kidney cancer, lung cancer, small intestine cancer, bone cancer, prostate cancer, colorectal cancer, breast cancer, bowel cancer, cervical cancer, ovarian cancer, lymphoma, nasopharyngeal carcinoma, adrenal tumor, bladder tumor, non-small cell lung cancer, brain glioma, endometrial cancer, fibrosarcoma, myxosarcoma, liposarcoma mesothelioma, lymphoid malignancies, or pancreatic cancer.

143. Use of the nanobody according to any one of claims 1 to 7, the chimeric antigen receptor according to claim 8, the chimeric antigen receptor according to claim 36, the nucleic acid molecule according to claim 71, the expression vector according to claim 83, the engineered host cell according to claim 86, or the immunoconjugate according to claim 91 in the manufacture of a medicament for treating and/or preventing a BCMA-positive related disease.

144. The use according to claim 143, wherein the BCMA-positive related disease comprises multiple myeloma, acute myeloid leukemia, chronic myeloid leukemia, chronic lymphocytic leukemia, acute lymphocytic leukemia, diffuse large B-cell lymphoma, Hodgkin's lymphoma, non-Hodgkin's lymphoma, gastric cancer, liver cancer, kidney cancer, lung cancer, small intestine cancer, bone cancer, prostate cancer, colorectal cancer, breast cancer, bowel cancer, cervical cancer, ovarian cancer, lymphoma, nasopharyngeal carcinoma, adrenal tumor, bladder tumor, non-small cell lung cancer, brain glioma, endometrial cancer, fibrosarcoma, myxosarcoma, liposarcoma mesothelioma, lymphoid malignancies, or pancreatic cancer.

145. Use of the nanobody according to any one of claims 1 to 7, the chimeric antigen receptor according to claim 8, the chimeric antigen receptor according to claim 36, the nucleic acid molecule according to claim 71, the expression vector according to claim 83, the engineered host cell according to claim 86, the immunoconjugate according to claim 91, or the pharmaceutical composition according to claim 116 in the manufacture of a biological agent for treating and/or preventing a BCMA-positive related disease.

146. The use according to claim 145, wherein the BCMA-positive related disease comprises multiple myeloma, acute myeloid leukemia, chronic myeloid leukemia, chronic lymphocytic leukemia, acute lymphocytic leukemia, diffuse large B-cell lymphoma, Hodgkin's lymphoma, non-Hodgkin's lymphoma, gastric cancer, liver cancer, kidney cancer, lung cancer, small intestine cancer, bone cancer, prostate cancer, colorectal cancer, breast cancer, bowel cancer, cervical cancer, ovarian cancer, lymphoma, nasopharyngeal carcinoma, adrenal tumor, bladder tumor, non-small cell lung cancer, brain glioma, endometrial cancer, fibrosarcoma, myxosarcoma, liposarcoma mesothelioma, lymphoid malignancies, or pancreatic cancer.

147. Use of the nanobody according to any one of claims 1 to 7, the chimeric antigen receptor according to claim 8, the chimeric antigen receptor according to claim 36, the nucleic acid molecule according to claim 71, the expression vector according to claim 83, the engineered host cell according to claim 86, the immunoconjugate according to claim 91, or the pharmaceutical composition according to claim 116 for treating and/or preventing a BCMA-positive related disease.

148. The use according to claim 147, wherein the BCMA-positive related disease comprises multiple myeloma, acute myeloid leukemia, chronic myeloid leukemia, chronic lymphocytic leukemia, acute lymphocytic leukemia, diffuse large B-cell lymphoma, Hodgkin's lymphoma, non-Hodgkin's lymphoma, gastric cancer, liver cancer, kidney cancer, lung cancer, small intestine cancer, bone cancer, prostate cancer, colorectal cancer, breast cancer, bowel cancer, cervical cancer, ovarian cancer, lymphoma, nasopharyngeal carcinoma, adrenal tumor, bladder tumor, non-small cell lung cancer, brain glioma, endometrial cancer, fibrosarcoma, myxosarcoma, liposarcoma mesothelioma, lymphoid malignancies, or pancreatic cancer.
